(19) **Europäisches Patentamt**
**European Patent Office**
**Office européen des brevets**

(11) **EP 3 067 092 A1**

(12) **EUROPEAN PATENT APPLICATION**
published in accordance with Art. 153(4) EPC

(43) Date of publication:
**14.09.2016 Bulletin 2016/37**

(51) Int Cl.:
**A61N 5/067** (2006.01)

(21) Application number: **14860490.3**

(22) Date of filing: **06.11.2014**

(86) International application number:
**PCT/JP2014/079420**

(87) International publication number:
**WO 2015/068758 (14.05.2015 Gazette 2015/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **07.11.2013 JP 2013231496**

(71) Applicant: **Arai Medphoton Research Laboratories, Corporation**
**Kawasaki-shi, Kanagawa 211-0063 (JP)**

(72) Inventors:
• **ARAI, Tsunenori**
  **Kawasaki-shi**
  **Kanagawa 211-0063 (JP)**
• **ITO, Arisa**
  **Kawasaki-shi**
  **Kanagawa 211-0012 (JP)**
• **KIMURA, Takehiro**
  **Tokyo 160-8582 (JP)**

(74) Representative: **Grünecker Patent- und Rechtsanwälte**
**PartG mbB**
**Leopoldstraße 4**
**80802 München (DE)**

(54) **MEDICAL INSTRUMENT AND LIGHT-RAY TREATMENT DEVICE**

(57)    Provided are a medical instrument capable of operating the entire treatment target site with a single irradiation by being provided with an irradiation range that can cover the entire treatment target site, and a light-ray treatment device. The medical instrument 30 is inserted into a living body and performs light irradiation on the treatment target site to treat the site. The medical instrument 30 comprises a lengthy medical instrument body 41 having at least one of lumens 42 and 43, and in the medical instrument body 41, lengthy light diffusers 44 and 45, and a lengthy reflector 46 having a reflection surface for reflecting light, each being inserted so as to be extended along the longitudinal direction of the medical instrument body 41. The medical instrument body 41 comprises, at least on a portion of the outer wall thereof, a transparent outer wall portion 41o that is optically transparent and extended along the longitudinal direction. The light diffusers 44 and 45 comprise a irradiation section 44 for irradiating light on the treatment target site and a light receiving section 45 for receiving light from the treatment target site, the light diffusers 44 and 45 being arranged so as to face the reflection surface and the transparent outer wall portion in a position between the reflection surface and the transparent outer wall portion.

FIG. 6

**Description**

TECHNICAL FIELD

The present invention is related to a medical instrument that comprises a light diffuser for irradiating light on a treatment target site and is used by being inserted into a living body, and a light-ray treatment device including the same.

BACKGROUND ART

Tachyarrhythmia is a type of arrhythmia that is caused by a conduction of abnormal excitation to a normal cardiac muscle tissue or formation of a turning circuit (a reentry circuit) of electrical excitation in a cardiac muscle tissue. Cardiac excitation is usually controlled to a normal rate (the sinus rhythm) by excitation from the sinoatrial node. In the case of tachyarrhythmia, however, the heartbeat is maintained at a rate faster than the sinus rhythm due to abnormal excitation conducted from some part of the cardiac tissue. The reentry circuit refers to a region where normal electrical excitation is not conducted but circles in a circuit shape by the presence of a conduction impaired site or the like in the cardiac muscle tissue.

Since it is difficult to identify a lesion in the treatment of arrhythmia, a conduction block for blocking an abnormal electrical signal needs to be set as a linear line. When the two pulmonary veins are collectively isolated, good results are obtained.

The treatment for tachyarrhythmia is commonly performed by thermally damaging an abnormal site by radio frequency ablation using a catheter. Instead of using the radio frequency ablation, studies on clinical application of a photodynamic therapy (PDT, also referred to as a "photochemical therapy") to tachyarrhythmia have been conducted.

The photodynamic therapy is a treatment method, in which a photosensitive substance is administered by intravenous injection or the like and, in a state that the photosensitive substance is distributed in a target tissue, a light beam such as a laser light is irradiated to induce a photosensitized reaction by the photosensitive substance, light, and oxygen, thereby causing necrosis in cells of the target tissue by this photosensitized reaction.

A known laser treatment system performing the photodynamic therapy comprises a laser catheter and a PDT device body that emits a laser light to the laser catheter and detects return light from the laser catheter (e.g., Patent Literature 1).

The laser catheter of Patent Literature 1 is constituted by comprising an optical fiber inserted in a catheter tube along the extension direction thereof and an optical window optically connected to a tip of the optical fiber at the foremost and outermost portion of the catheter tube.

The optical window transmits irradiation light emitted from the tip of the optical fiber and also converges fluorescence emitted from a PDT agent at the tip of the optical fiber.

The laser catheter of Patent Literature 1 having such a configuration can perform the photodynamic therapy by contacting the tip of the laser catheter with a treatment target tissue.

However, the laser catheter of Patent Literature 1 is, similar to a catheter for radio frequency ablation, configured to irradiate a laser from the tip of the catheter tube.

Thus, when arrhythmia is treated by using the laser catheter of Patent Literature I, as in the case of the radio frequency catheter ablation, irradiation is performed only in one place at a time and it is still necessary to connect each irradiation point in order to set a line for blocking an abnormal electrical signal.

The laser catheter of Patent Literature 1 had a limitation in a range where laser irradiation can be performed, thus, in order to create an abnormal electrical conduction blocking line, it was necessary to shift the irradiation range sequentially to obtain a lineally connected treatment area.

On the other hand, as a hand-held resection device used for treating arrhythmia by forming a conduction block around the pulmonary vein, a device forming photo ablation by infrared ray irradiation and the like is known (e.g., Patent Literature 2).

The resection device in Patent Literature 2 is used in an epicardial approach performed during surgical procedures in which the thorax of a patient is opened and in an endocardial approach performed during valve replacement procedures in which the thorax of a patient is opened to expose the cardiac muscles, and comprises a light diffuser in a loop-shaped tip portion in a manner that the light diffuser is extend along the longitudinal direction of the tip portion.

According to the resection device in Patent Literature 2, linearly connected ablation can be formed as long as the length of the light diffuser, thus the abnormal electrical conduction blocking line can be formed more easily.

CITATION LIST

PATENT LITERATURE

PATENT LITERATURE 1: JP 2012-147937 A
PATENT LITERATURE 2: JP 2008-501441 A

SUMMARY OF INVENTION

TECHNICAL PROBLEM

However, the resection device in Patent Literature 2 comprises the light diffuser having a length of about 4cm, and this length only accounts for a portion of the length of the abnormal electrical conduction blocking line to be formed, which is about 10cm. Thus, after all, it was necessary to repeatedly perform a procedure of sequentially shifting the light diffuser and a procedure of irradiating light.

Further, when the abnormal electrical conduction blocking line was formed by the resection device in Patent Literature 2, there was no section provided for measuring in real time information on a degree of each ablation formed by the light diffuser.

In surgical operations and the like in which the thorax of a patient is opened, the information on a degree of ablation formed by the light diffuser could be judged in real time by visual inspection and the like by an operator. However, when the abnormal electrical conduction blocking line was formed by a laser catheter intravenously inserted into the heart, it was impossible for an operator to judge the information on a degree of ablation formed by the light diffuser directly by visual inspection and the like.

Especially, in the photodynamic therapy for arrhythmia, a conduction pathway of the electrical signal is tracked down by performing an electrophysiologic test and a treatment for blocking this pathway is performed instantly. The therapy is operated under the fully monitored condition while performing the electrophysiologic test and involves an instantaneous decision of the treatment, thus it is necessary to achieve an electrical conduction block in the cardiac muscle tissue by an instantaneous treatment.

Although whether the electrical conduction block is achieved or not can be judged by the electrophysiologic test, there is no way of informing an operator in real time of the information on a degree of ablation caused to the tissue each moment by light irradiation from a laser, and this has been a barrier for clinical application of the photodynamic therapy for arrhythmia.

The present invention has been made in view of the above-mentioned problems and it is an object of the present invention to provide a medical instrument for irradiating light on a treatment target site, capable of operating the entire treatment target site with a single irradiation by being provided with an irradiation range that can cover the entire treatment target site, and a light-ray treatment device.

It is another object of the present invention to provide a medical instrument capable of performing a photodynamic therapy and measuring a progress of the photodynamic therapy in a single device, and a light-ray treatment device.

It is another object of the present invention to provide a medical instrument capable of forming an abnormal electrical conduction blocking line for arrhythmia with a single irradiation by being provided with an irradiation range that can cover the entire abnormal electrical conduction blocking line, and a light-ray treatment device.

SOLUTION TO PROBLEM

According to the medical instrument of the present invention, the above-mentioned problems can be solved by the medical instrument that is inserted in a living body and irradiates light on a treatment target site for treating the site, comprising: a lengthy medical instrument body having at least one lumen; and, in the medical instrument body, a lengthy light diffuser for irradiation for irradiating light on the treatment target site; a lengthy light diffuser for receiving light for receiving light from the treatment target site; and a lengthy reflector having a reflection surface for reflecting light, each being inserted so as to be extended along the longitudinal direction of the medical instrument body, wherein: the medical instrument body comprises, at least on a portion of an outer wall thereof, a transparent outer wall portion that is optically transparent and extended along the longitudinal direction; and the light diffuser comprises an irradiation section that irradiates light on the treatment target site and a light receiving section that receives light from the treatment target site and is arranged so as to face the reflection surface and the transparent outer wall portion in a position between the reflection surface and the transparent outer wall portion.

In this configuration, the medical instrument body is provided with the lengthy light diffuser comprising the irradiation section that irradiates light on the treatment target tissue and the light receiving section that receives light from the treatment target tissue, and the lengthy reflector, each being extended along the longitudinal direction of the medical instrument body, thus light can be irradiated in a long range and a wide irradiation range can be secured. Accordingly, it becomes unnecessary for an operator to repeatedly perform irradiation and shifting of the medical instrument in order to perform irradiation on the entire treatment target site, and becomes possible to perform an operation in a wide range with a single irradiation treatment, thus allowing a rapid treatment.

In particular, the medical instrument can be also suitably applied to the photodynamic therapy for arrhythmia, in which, while the conduction pathway of the electrical signal is being tracked down, the treatment for blocking this pathway needs to be performed instantly.

Further, the medical instrument comprises the light receiving section, thus, monitoring the progress of the treatment in real time, monitoring the operating conditions of the treatment device to confirm, for example, whether irradiation is properly performed according to the setting, and the like can be performed simultaneously.

The inventors of the present invention have found, as a result of their earnest studies, that the progress of the photodynamic therapy can be monitored by measuring fluorescence when excitation light is irradiated. This finding and the present invention make it possible to monitor in real time the progress of the photodynamic therapy from measurement values of return fluorescence received by the light diffuser for receiving light.

Further, the irradiation section and the light receiving section are arranged so as to face the reflection surface and the transparent outer wall portion in the position between the reflection surface and the transparent outer wall portion, thus, by the reflection surface, light irradiation by the irradiation section can be focused on a proper site and, in the same time, fluorescence can be efficiently received when the light is irradiated.

Further, the irradiation section, the light receiving section, and the reflector can be compactly disposed, thereby enabling to achieve the medical instrument having a small diameter.

Since both the light diffuser and the reflector are formed in a lengthy shape, light that is emitted through a long continuing distance can be received through a long continuing distance, thus the progress of the treatment can be monitored based on an average value of the entire irradiation range. As such, even when the progress of the treatment is particularly too advanced or delayed only in some portions of the irradiation range, it is still possible to monitor the treatment progress based on the actual condition thereof, instead of monitoring based on extreme values.

In this configuration, the light diffuser may comprise a lengthy light diffuser for irradiation constituting the irradiation section, extended along the longitudinal direction of the medical instrument body, and a lengthy light diffuser for receiving light constituting the light receiving section, formed as a different body from the light diffuser for irradiation and extended along the longitudinal direction of the medical instrument body, and the light diffuser for irradiation and the light diffuser for receiving light may be adjacently arranged so as to face the reflection surface and the transparent outer wall portion in a position between the reflection surface and the transparent outer wall portion.

In this manner, the light diffuser is constituted of the lengthy light diffuser for irradiation and the lengthy light diffuser for receiving light formed as a different body from the light diffuser for irradiation, thus the irradiation section and the light receiving section can be achieved with a simple configuration. Further, since the light diffuser for irradiation and the light diffuser for receiving light are separately constituted, controls of the irradiation section and the light receiving section can be simplified.

Further, the light diffuser for irradiation and the light diffuser for receiving light are adjacently arranged so as to face the reflection surface and the transparent outer wall portion in a position between the reflection surface and the transparent outer wall portion, thus the medical instrument can be made compact with a small diameter.

In this configuration, the medical instrument may comprise a tip portion composed of a loop portion that is wound along over one circumference in a helical shape when an external force is not applied thereto, and a bent portion bent at an end portion of the loop portion. The light diffuser for irradiation and the light diffuser for receiving light may be extended in a region, of the tip portion, that is wound along over one circumference in the helical shape.

Having such a configuration makes it possible to perform light irradiation in an annular shape such as seen in the abnormal electrical conduction blocking line created in the treatment of arrhythmia with a single irradiation treatment. Thus, for the purpose of creating an annular line, it is not necessary to connect each irradiation point to form an irradiation line by sequentially shifting an irradiation probe of the medical instrument, thereby allowing a rapid treatment.

In this configuration, the reflection surface may face to the outer peripheral side of the helical shape and the light diffuser for irradiation and the light diffuser for receiving light may be positioned at the outer peripheral side of the reflection surface.

With such a configuration, an irradiation mode of the light diffuser for irradiation is converted to a mode in which light is emitted from an annular body toward the outer peripheral side, and light is easily irradiated, for example, from the inside of the blood vessel to the whole periphery of the inner wall of the blood vessel.

In this configuration, a plurality of electrodes may be arranged around the tip portion at predetermined intervals in an extension direction of the tip portion.

With such a configuration, the plurality of the electrodes are arranged along the extension direction of the tip portion, thus it becomes possible to monitor, in a well-balanced manner, whether the entire region of the tip portion is properly abutted with and arranged to the treatment target site by monitoring a signal from the electrodes.

Further, by using the medical instrument of the present invention, there is no need to separately use an electrode catheter in the treatment of arrhythmia and the like, and the single device can do the job of three, namely, light irradiation and measurement of the progress of the treatment by the light diffuser, and potential measurement by the electrodes.

In this configuration, a shape memory member having shapes of the loop portion and the bent portion, when the external force is not applied thereto, may be stored inside the tip portion. The medical instrument body may comprise a plurality of lumens extended along the extension direction of the medical instrument body, wherein the light diffuser for irradiation and the light diffuser for receiving light, lead wires of the electrodes, and the shape memory member may be stored in the different lumens.

With such a configuration, the light diffuser for performing light irradiation and reception, and other constituent elements can be stored in the different lumens, thus optical paths of light that is irradiated and received by the light diffuser can be prevented from being interfered by the other constituent elements.

In this configuration, the plurality of the lumens may be divided into an inner peripheral side lumen arranged on the inner peripheral side of the helical shape and an outer peripheral side lumen arranged on the outer peripheral side. The shape memory member and a plurality of the lead wires connected to the plurality of the electrodes may be stored in the inner peripheral side lumen, while, in the outer peripheral side lumen, the reflector may be arranged adjacent to the inner peripheral side lumen, and the light diffuser for irradiation and the light diffuser for receiving light may be arranged on the outer peripheral side of the reflector.

With such a configuration, the light diffuser for performing light irradiation and reception is located on the outer peripheral side of the helical shape and other constituent elements are located on the inner peripheral side, thus irradiation of light toward the outside of the helical shape and reception of light from the outside of the helical shape can be ensured.

Thus, it becomes possible to perform light irradiation that is focused on the treatment target site, and, in the same time, a site other than the treatment target site can be prevented from receiving light irradiation. As a result, the amount of irradiation to the blood that is not the treatment target is reduced and damages on the blood such as hemolysis can be suppressed.

In this configuration, the plurality of the lumens may be composed of a shape memory member lumen for storing the shape memory member, a lead wire lumen for storing the plurality of the lead wires connected to the plurality of the electrodes, a lumen for light diffuser for irradiation for storing the light diffuser for irradiation, and a lumen for light diffuser for receiving light for storing the light diffuser for receiving light, arranged adjacent to the lumen for light diffuser for irradiation in the circumferential direction of the medical instrument body. The lumen for light diffuser for irradiation and the lumen for light diffuser for receiving light may be arranged on the outer peripheral side of the medical instrument body.

With such a configuration, the light diffuser for performing light irradiation and reception is located on the outer peripheral side of the helical shape and other constituent elements are located on the inner peripheral side, thus irradiation of light toward the outside of the helical shape and reception of light from the outside of the helical shape can be ensured.

In this configuration, the medical instrument may be a laser catheter used for the photodynamic therapy for arrhythmia.

Further, a light-ray treatment device may comprise: the medical instrument; a light source that projects excitation light to the light diffuser of the medical instrument; a detection section that detects fluorescence received from the light diffuser of the medical instrument; and an arithmetic section that receives signals of the detected excitation light and fluorescence from the detection section and calculating the progress of the light-ray treatment performed by the medical instrument based on the signals.

EFFECTS OF INVENTION

According to the present invention, the medical instrument body is provided, in its inside, with the lengthy light diffuser comprising the irradiation section that irradiates light on the treatment target site and the light receiving section that receives light from the treatment target site, and the lengthy reflector, each being extended along the longitudinal direction of the medical instrument body. Thus, light can be irradiated in a long range and a wide irradiation range can be secured. Accordingly it becomes unnecessary for an operator to repeatedly perform irradiation and shifting of the medical instrument in order to perform irradiation on the entire treatment target site, and becomes possible to perform an operation in a wide range with a single irradiation treatment, thus allowing a rapid treatment.

In particular, the medical instrument can be also suitably applied to the photodynamic therapy for arrhythmia, in which, while the conduction pathway of the electrical signal is being tracked down, the treatment for blocking this pathway needs to be performed instantly.

Further, the medical instrument is provided with the light receiving section, thus monitoring of the treatment progress can be simultaneously performed in real time.

The inventors of the present invention have found, as a result of their earnest studies, that the progress of the photodynamic therapy can be monitored by measuring fluorescence when excitation light is irradiated. This finding and the present invention make it possible to monitor in real time the progress of the photodynamic therapy by measurement values of return fluorescence received by the light diffuser for receiving light.

Further, the irradiation section and the light receiving section are arranged so as to face the reflection surface and the transparent outer wall portion in the position between the reflection surface and the transparent outer wall portion, thus, by the reflection surface, light irradiation by the irradiation section can be focused on a proper site and, in the same time, fluorescence can be efficiently received when the light is irradiated.

Further, the irradiation section, the light receiving section, and the reflector can be compactly disposed, thereby enabling to achieve the medical instrument having a small diameter.

Since both the light diffuser and the reflector are formed in a lengthy shape, light that is irradiated through a long continuing distance can be received through a long continuing distance, thus the treatment progress can be monitored based on a value averaged over the entire irradiation range. As such, even when the progress of the treatment is particularly too advanced or delayed only in some portions of the irradiation range, it is still possible to monitor the treatment progress based on the actual condition thereof, instead of monitoring based on extreme values.

BRIEF DESCRIPTION OF DRAWINGS

FIG. 1 is a block diagram illustrating an approximate configuration of a photodynamic therapy device comprising a laser catheter and a treatment device according to one embodiment of the present invention.

FIG. 2 is an explanatory diagram illustrating a concept of a PBI (Photosensitizerbleaching index).

FIG 3 is a graph showing a change in fluorescence detector output over time in an extracellular photodynamic therapy to which the laser catheter according to one embodiment of the present invention is applied.

FIG. 4 is a schematic explanatory diagram illustrating a state in which the laser catheter according to one embodiment of the present invention is applied to a treatment for arrhythmia.

FIG. 5 is an explanatory diagram illustrating a head portion of the laser catheter according to one embodiment of the present invention.

FIG. 6 is a sectional view taken along a line B-B of Fig. 5.

FIG. 7 is an explanatory sectional view of a modified example of the head portion of the laser catheter.

FIG. 8 is an explanatory longitudinal sectional view illustrating the head portion of the laser catheter according to one embodiment of the present invention.

FIG. 9 is a schematic explanatory diagram illustrating a light diffuser for irradiation according to one embodiment of the present invention.

FIG. 10 is an explanatory cross-sectional view illustrating a head portion of a laser catheter according to another embodiment of the present invention.

FIG. 11 is an explanatory diagram illustrating a device configuration of a fluorescence measuring system in verification test 1 (Example 1, Comparative example 1).

FIG. 12 is a graph showing spectra measured in Example 1, Comparative example 1, and Control 1 in verification test 1.

FIG. 13 is a graph showing a change in fluorescence intensity over time in a cardiac muscle tissue immersed in an agent solution (Example 1) at a wavelength of 704nm.

FIG. 14 is a graph showing a fluorescence spectrum of a talaporfin sodium solution (Control 2).

FIG. 15 is an explanatory diagram illustrating a device configuration of a fluorescence measuring system in verification test 2 (Example 1).

FIG. 16 is a graph showing transmission spectra of a polarization beam splitter 65' and a dichroic mirror 63'.

FIG. 17 is a graph showing a fluorescence spectrum at a light irradiation start time in verification test 2.

FIG. 18 is a graph showing detection values of return fluorescence at a wavelength of 650 to 900nm after 3 minutes from starting the light irradiation in verification test 2.

FIG. 19 is a graph showing a change in fluorescence intensity over time at a wavelength of 710nm in verification test 2.

DESCRIPTION OF EMBODIMENTS

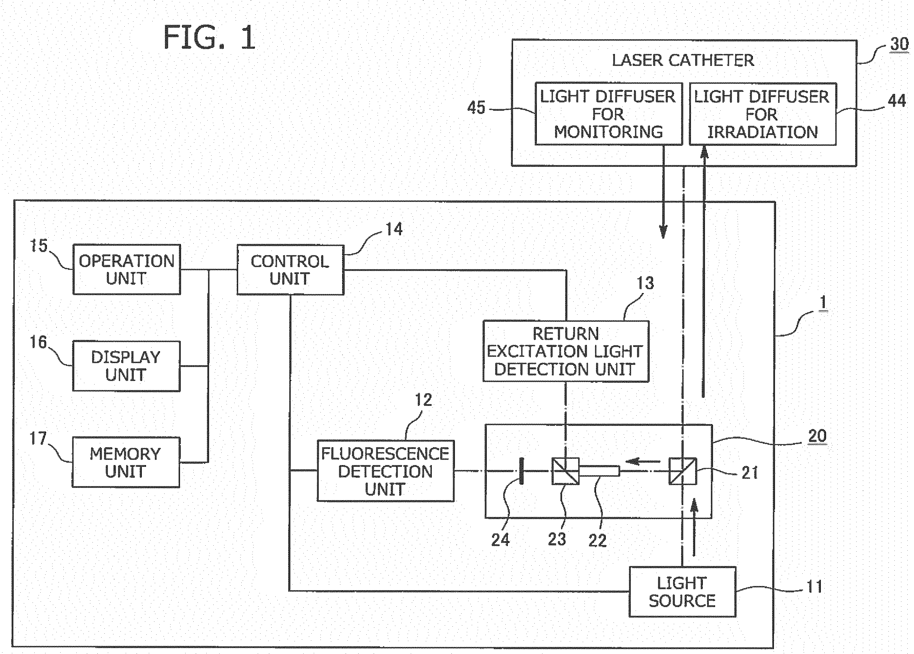

Hereinafter, a laser catheter 30 according to one embodiment of the medical instrument of the present invention will be described with reference to Fig. 1 to Fig. 9.

It is noted that, in the present embodiment, the laser catheter 30 is described as the medical instrument, however, it is not limited thereto, and any medical instruments that are introduced into a treatment target tissue in a living body, such as sheath, endoscope, venous conduit, arterial conduit, bronchoscope, cystoscope, culpascope, colonoscopy, trocar, laparoscopy, and other medical tubes, may be included.

Further, in the embodiment of the description, an example of using the medical instrument of the present invention in the treatment of arrhythmia, in which an abnormal electrical conduction blocking line is created by the photodynamic therapy, is described. However, it is not limited thereto, and the medical instrument can be used for various treatments that include, for example, the photodynamic therapy for cancer in the pancreas and the biliary tract, performed under an endoscope using a small-diameter endoscope with an extremely fine shape, such as a biliary endoscopy (outer diameter of 1 to 3mm) and a pancreatic endoscopy (outer diameter of 1 to 2.5mm).

The medical instrument of the present invention can be used for any medical conditions where a medical instrument comprising a light radiation probe represented by the laser catheter 30 can be used. For example, it can be used for the photodynamic therapy for cancer, infectious diseases, arteriosclerosis, etc., a treatment of thrombosis using a laser catheter, and the like. Further, it can be used in any cases where laser irradiation and laser measurement are performed.

In the description, in a state that the laser catheter 30 is inserted in a living body, a proximal side of the laser catheter 30, etc. refers to an outer side of the living body, that is, an operator side, while a distal side of the laser catheter 30 refers to a tip side of the catheter portion inserted in the living body, that is, a treatment target tissue side.

The laser catheter 30 of the present embodiment is, as shown in Fig. 1, used together with a treatment device 1. A combination of the laser catheter 30 and the treatment device 1 corresponds to a light-ray treatment device in embodiments.

The treatment device 1 is a device for carrying out an extra-cellular photodynamic therapy (Extra-cellular PDT) that is performed by sufficiently distributing a photosensitive substance (hereinafter referred to as a PDT agent) in the extracellular interstitium and the blood vessel inside of the treatment target tissue as a treatment target site in embodiments. The treatment device 1 performs a photodynamic therapy by projecting light for photodynamic therapy to a light diffuser for irradiation of the laser catheter 30, and, in the same time, receives return fluorescence from a light diffuser for monitoring, thereby calculating an index value of information on a treatment progress in real time during the treatment and displaying it on a screen. An operator can speculate a degree of ablation caused to the treatment target tissue by light irradiation each moment while referring to the index value of information on the treatment progress displayed on the screen in real time during the treatment.

The extra-cellular PDT of the present embodiment is carried out in a circumstance where a PDT agent is distributed to an extra-cellular region of a living body, that is, an extracellular fluid and/or a transcellular fluid, and also continuously supplied thereto by vascular permeability.

In the present embodiment, the treatment device 1 is used for treating arrhythmia, however, it may be used for any extra-cellular PDT that is performed by sufficiently distributing a PDT agent in the extracellular interstitium and the blood vessel inside of the treatment target tissue, thus it can be also used for other treatments including the photodynamic therapy for infectious diseases. As long as a PDT agent and oxygen are sufficiently and continuously supplied to the extracellular interstitium and the blood vessel inside of the treatment target tissue, the treatment device 1 can be used for cancer treatments (including gastroenterology division, respiratory system division, brain surgery division, and dermatology division) in which a PDT agent is accumulated inside of cells in a treatment target tissue, a treatment of arteriosclerosis, and the like. Further, it can be also used for angioplasty.

A type of arrhythmia treated by using the treatment device 1 of the present embodiment includes arrhythmia that is caused by the presence of an abnormal electrical conduction site or an abnormal excitation generation site, especially, tachyarrhythmia, and any types of tachyarrhythmia that are conventionally treated by a radio frequency ablation therapy are included. Specific examples thereof include atrial fibrillation (AF) such as paroxysmal AF, persistent AF, and permanent AF; atrial flutter (AFL); and paroxysmal supraventricular tachycardia such as atrioventricular reciprocating tachycardia (AVRT), atrioventricular nodal reentrant tachycardia (AVNRT), and atrial tachycardia (AT).

Further, as infectious diseases, MRSA infection, gingivitis, periodontitis, peri-implantitis, herpes, canker sores, and candidiasis are included.

A PDT agent used in the present embodiment is a photosensitive substance.

In the present embodiment, unlike a photodynamic therapy for cancer, it is not necessarily to accumulate an agent in a tissue, thus a treatment is started shortly after the administration of the agent by setting Drug Light interval, an interval from administration of agent to light irradiation, to a short time of about several minutes to several tens of minutes. Accordingly, it is preferred to use a water-soluble photosensitive substance that is rapidly distributed in the interstitium after the administration by intravenous injection and the like, and rapidly discharged.

Examples of such an agent include a chlorin-based agent having a chlorin skeleton, such as ATX-S10 (670nm) (an iminochlorin aspartic acid derivative, (Oriental Menthol Industry Ltd., rights were transferred to Photochemical Co., Ltd. in 2000, JP-A-6-80671), NPe6 (664nm) (talaporfin sodium, Laserphyrin (registered trademark), mono-L-aspartyl chlorin e6, JP Pat. No. 2961074), mTHPC (652nm), SnET2 (660nm) (tin etiopurpurin, Miravant Medical Technologies), AIPcS (675nm) (chloro aluminium sulphonated phthalocyanine), BPD-MA (690nm) (benzoporphyrin derivative monoacid ring A, QLT Inc.), Lu-tex (732nm) (Lutetium Texaphyrin), and the like. Of these, talaporfin sodium is preferable.

<<Principle and reaction mechanism of extra-cellular PDT for arrhythmia >>

In a photodynamic therapy for arrhythmia of the present embodiment, the therapy utilizes a photosensitizing reaction occurring in the early stage after the administration of a PDT agent, that is, at the timing when the PDT agent is distributed extracellularly (in an interstitial space) instead of intracellularly.

After the PDT agent is administered by intravenous injection and the like, the PDT agent is initially distributed in the extracellular interstitium and in the blood vessel at a high concentration. During this period, an extracellular region is in a state of being continuously supplied with the PDT agent and oxygen at a sufficient level by the bloodstream all the time. The PDT agent is supplied to the interstitium from the blood vessel by permeability.

When laser light is irradiated on a target site via a catheter by setting Drug Light interval from administration of agent to light irradiation to a short time of about several minutes to several tens of minutes, light is supplied at a sufficient level by the laser light while the PDT agent and oxygen are supplied at a sufficient level by the bloodstream. As a result, a photosensitizing reaction by the PDT agent, light, and oxygen occurs.

In the photosensitizing reaction, the PDT agent is excited by light beam irradiation. Energy of the excited PDT agent is transferred to oxygen in the extracellular region and generates active singlet oxygen (active oxygen).

The total production amount of singlet oxygen is an irradiation-time integration value of an amount of singlet oxygen per unit time.

When excited singlet oxygen is produced, a protein bound to the PDT agent is destroyed, the excited singlet oxygen is converted to triplet ground-state oxygen, and agent bleaching occurs by a strong oxidizing power of this singlet oxygen. As a therapeutic effect, elimination of electrical conduction and necrosis in the cardiac muscle cells caused by oxidative damage on ion channels and cell membranes are obtained.

The term "agent bleaching" in this description refers to a phenomenon that a photosensitive substance as the PDT agent is destroyed by singlet oxygen. A quantity of agent bleaching is proportional to a changing amount of fluorescence of the PDT agent per unit time.

<<PBI (Photosensitizer bleaching index)>>

In the present embodiment, a PBI (Photosensitizer bleaching index) is used as an indicator value of a therapy depth of the extra-cellular PDT.

In an extracellular photosensitizing reaction occurring in the extra-cellular PDT, the PDT agent is sufficiently supplied by the bloodstream during the treatment, thus it can be assumed that time integration of a value of agent bleaching corresponds to the total amount of agent bleaching up to that time point, that is, the total production amount of singlet oxygen serving as a therapeutic effect factor.

Therefore, in the present embodiment, as an indicator corresponding to the total amount of the bleaching, the PBI represented by the following formula 1 is used.

[Mathematical 1]

$$PBI = (fluo(0) - fluo(t_d))t_d \qquad \text{(formula 1)}$$

It is noted that, in formula 1, the following abbreviations are used:

fluo(t): return fluorescence intensity at a time point t
$t_d$: light irradiation time [s]

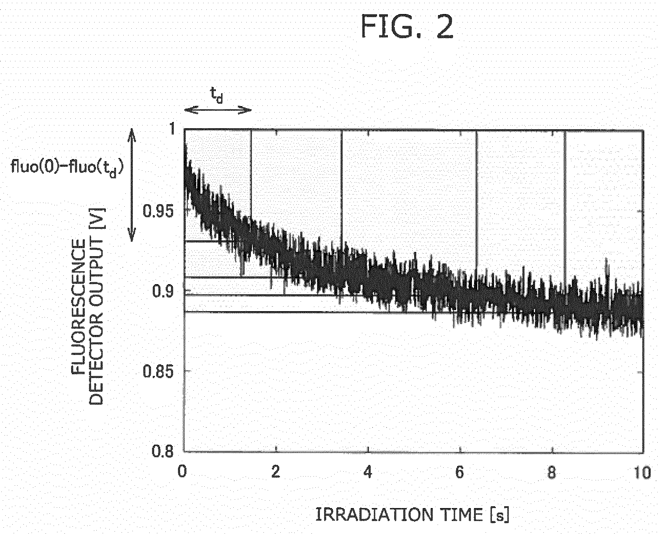

A concept of the PBI is described based on Fig. 2. FIG. 2 is a graph showing a change of return fluorescence intensity over time in an extracellular photosensitizing reaction, where a plurality of rectangles, representing a concept of the PBI, are drawn in superposition.

The PBI is a value obtained by multiplying a difference obtained by subtracting fluorescence intensity at a light irradiation start time to from fluorescence intensity at a light irradiation time $t_d$ indicating a measurement time by the light irradiation time $t_d$ up to the measurement time.

In Fig. 2, sides of the plurality of the rectangles in a lateral axis direction correspond to the light irradiation time $t_d$ up to the measurement time.

Further, at the light irradiation start time to, that is, at a time 0 in Fig. 2, light irradiation is not initiated yet, thus the agent bleaching doesn't occur and return fluorescence intensity fluo(t) shows the highest value. Subsequently, after a time 0, light irradiation is performed, and as the measurement time $t_d$ passes, the agent bleaching proceeds and the fluo(t) decreases.

Thus, a side of the each rectangle in a longitudinal axis direction corresponds to a difference obtained by subtracting fluorescence intensity at the light irradiation start time to from fluorescence intensity at the light irradiation time $t_d$ indicating the measurement time.

Consequently, the PBI corresponds to an area of the each rectangle in Fig. 2.

In formula 1, it is assumed that oxygen and the PDT agent are sufficiently supplied at a constant rate by permeability from the blood vessel to the interstitium. Thus, a rate-determining step is an amount of light energy to be charged.

Further, the measurement values of fluorescence include both measurement values of fluorescence of the PDT agent from the blood and the interstitium, and an abundance ratio of the fluorescence therebetween is assumed to be constant.

In the extracellular photosensitizing reaction, it is assumed that the singlet oxygen production amount is proportional to the amount of light energy to be charged, thus an estimated value of necrosis depth by the extracellular photosensitizing reaction, that is, an estimated therapy depth $d_{nec}$, depends on light distribution in the depth direction.

Accordingly, the estimated therapy depth $d_{nec}$ can be expressed as a logarithm of the PBI in the following formula 2, obtained by modifying a general formula indicating an absorption coefficient, $E_{(d)} = I_0 t \cdot \exp(-\mu_{cff} d)$.

[Mathematical 2]

$$d_{nec} = 1/\mu_{eff} \ln(k(t)\, I_o\, PBI/E)$$
$$= 1/\mu_{eff} \{\ln(PBI) + \ln(k(t)\, I_0/E)\} \qquad \text{(formula 2)}$$

It is noted that, in formula 2, the following abbreviations are used:

$d_{nec}$: ablation depth [mm]
$\mu_{eff}$: attenuation coefficient [/mm]
k(t): constant that varies with time 1/{fluo(0)-fluo(t)}
$I_0$: light intensity on tissue surface [mW]
E: light energy at given depth [J]

«Correction of PBI»

The PBI obtained by formula 1 can be used to provide a highly useful indicator further matching to an actual state by calculating a corrected PBI that corrects the PBI.

Methods for correction can be divided into correction methods that utilize a temporally changing fluorescence intensity waveform itself and correction methods that utilize physiological information on a tissue and the like obtained by other methods.

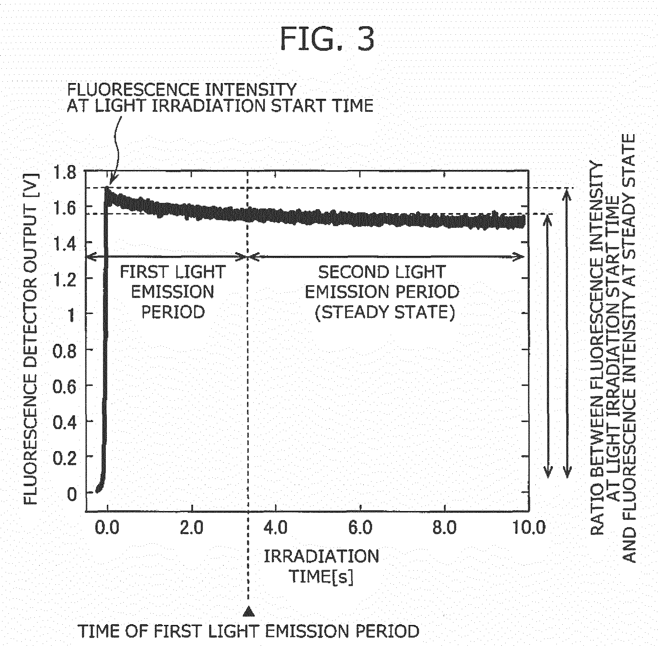

Among the correction methods that utilize a temporally changing fluorescence intensity waveform itself, the following three methods, 1, 2 and 3 are described based on a graph in Fig. 3. FIG. 3 is a graph showing a change of fluorescence detector outputs over time in the extra-cellular PDT.

As shown in Fig. 3, it is confirmed that, in the extra-cellular PDT, fluorescence intensity detected by a fluorescence detection portion 12 in Fig. 1 reaches a steady state some time after staring the light irradiation.

Thus, a time period from the start of light irradiation to the end of light irradiation can be divided into a first light emission period from the start of light irradiation until when fluorescent intensity reaches a steady state and a second light emission period having the steady state continued from the first light emission period until the end of light irradiation.

Correction method 1

A corrected PBI can be obtained by multiplying the PBI obtained by formula 1 by a fluorescence intensity output at the light irradiation start time.

The fluorescence intensity at the light irradiation start time reflects a concentration of the PDT agent, thus such correction makes it possible to obtain a corrected PBI that includes a concentration of the PDT agent distributed in the tissue.

Correction method 2

A ratio between the fluorescence intensity at the light irradiation start time and the fluorescence intensity at the steady state indicates a transition state in the first light emission period proceeding to the steady state.

The PBI indicates a reaction progress in the steady state. Thus, the PBI is corrected in accordance with a state in progress so as to reflect the photosensitizing reaction occurring in the transition state in the first light emission period, by using two indicators, namely a fluorescence intensity ratio between in the first light emission period and in the steady state and a fluorescence intensity ratio between at the light irradiation start time and in the steady state.

The first light emission period is different from the second light emission period having the steady state in a balance between supply of the PDT agent and oxygen by vascular permeability or the bloodstream and consumption of the PDT agent and oxygen by the photodynamic therapy.

Thus, according to the correction method 2, there can be obtained a corrected PBI that matches to an actual state of the first light emission period having the transition state, which is caused by being different from the steady state in a balance between supply of the PDT agent and oxygen by vascular permeability or the bloodstream and consumption of the PDT agent and oxygen by the photodynamic therapy.

Correction method 3

A corrected PBI can be obtained by dividing the PBI obtained by formula 1 by return excitation light intensity at the irradiation time of interest.

With such a correction, there can be obtained the corrected PBI that includes differences in optical characteristics among target tissues of the therapy.

In the correction methods that utilize physiological information on a tissue and the like obtained by other methods, a correction is made by using physiological information on a tissue obtained through measurement by other methods, information on systemic conditions of a patient obtained by general living-body monitoring devices, or known physiological numerical values related to a target tissue.

Examples of the physiological information, the information on systemic conditions, and the physiological numerical values include: oxygen concentrations such as $PaO_2$ (partial pressure of oxygen in arterial blood) and $SpO_2$ (endermic arterial oxygen saturation); hemoglobin contents such as a total hemoglobin content, an oxygenated hemoglobin content, and a deoxygenated hemoglobin content; a blood flow rate, a bloodstream quantity in each tissue; a vessel running density per unit volume; a total blood volume; temperature of a patient; temperature of a target tissue; and concentrations of serum proteins such as albumin, HDL (high-density lipoprotein), and LDL (low-density lipoprotein).

Of those, the oxygen concentrations are factors that affect reaction efficiency and oxygen supply. Further, the hemoglobin contents are factors that affect oxygen supply. The bloodstream quantity and the blood flow rate are factors that affect supply of the PDT agent and oxygen. The temperatures are a factor that affects reaction efficiency. The serum protein contents affect a supply quantity of the PDT agent since a serum protein binds to the PDT agent.

<<Configuration of treatment device 1>>

Next, the treatment device 1 of the present embodiment is described based on Fig. 1.

FIG. 1 is a block diagram illustrating an approximate configuration of the treatment device 1 of the present embodiment.

The treatment device 1 comprises a light source 11, an optical system 20, a fluorescence detection unit 12, a return excitation light detection unit 13, a control unit 14, an operation unit 15, a display unit 16, and a memory unit 17.

The light source 11 outputs excitation light that excites a PDT agent. A wavelength of the light output by the light source 11 is the same as a Q-band absorption wavelength of the PDT agent. Talaporfin sodium is used as the PDT agent in the present embodiment, thus as the light source 11, a device outputting light having a wavelength of 400 to 700nm, preferably approximately 660nm, is used. It is noted that when an agent other than talaporfin sodium is used as the PDT agent, a wavelength of light output from the light source 11 is not limited thereto.

As the light source 11, a laser device such as a semiconductor laser device, a light emitting diode, or a lamp light source is used.

The excitation light output from the light source 11 enters a light diffuser for irradiation 44 of the laser catheter 30 by the optical system 20. In this process, it is preferred that an input power of the light source 11 is 1000mW or less, preferably 500mW or less, and an output of the light diffuser for irradiation 44 of the laser catheter 30 as the whole length is 150mW/cm or less, preferably 75mW/cm or less. Having such a configuration can adjust intensity of light irradiation from the light diffuser for irradiation 44 to an appropriate level and prevent a region of penetration depth from becoming too wide.

The optical system 20 makes the excitation light emitted from the light source 11 incident into the light diffuser for irradiation 44 of the laser catheter 30 via a polarization beam splitter 21. Further, the optical system 20 extracts, from a light diffuser for monitoring 45 as a light diffuser for receiving light of the laser catheter 30, fluorescence emitted from the PDT agent that has been irradiated with the excitation light, and excitation light returning from the laser catheter 30, and makes them incident into the fluorescence detection unit 12 and the return excitation light detection unit 13, respectively.

It is noted that, in the present embodiment, the light diffuser for monitoring 45 only receives light, however it is not limited thereto, and the light diffuser for monitoring 45 may irradiate excitation light for measurement.

The optical system 20 comprises the polarization beam splitter 21, an optical fiber 22, a dichroic mirror 23, and a band pass filter 24.

The excitation light from the light source 11 enters the light diffuser for irradiation 44 of the laser catheter 30. A portion of the excitation light from the light source 11 is reflected on side end faces of an optical fiber 44f and a connector not illustrated, connecting the treatment device 1 and the laser catheter 30, and a tip portion of the laser catheter 30, and enters the polarization beam splitter 21 as return excitation light.

Further, fluorescence from the light diffuser for monitoring 45 of the laser catheter 30 also enters the polarization beam splitter 21.

The polarization beam splitter 21 reflects light made incident from the light diffuser for monitoring 45 of the laser catheter 30 and guides it into the dichroic mirror 23 via the optical fiber 22.

The dichroic mirror 23 reflects light with a wavelength of 680 to 690nm or less and transmits light with a wavelength of 680 to 690nm or more. By this feature, the dichroic mirror 23 transmits the fluorescence having a peak wavelength of approximately 710nm and guides it to the fluorescence detection unit 12 via the band pass filter 24, and reflects the return excitation light having a peak wavelength of approximately 663nm and guides it to the return excitation light detection unit 13.

The band pass filter 24 is a filter that transmits light having a wavelength of 710 ± 2nm.

Talaporfin sodium is used as the PDT agent in the present embodiment, thus the dichroic mirror 23 and the band pass filter 24 transmitting and reflecting light of the above-mentioned wavelengths are used, however, when an agent other than talaporfn sodium is used as the PDT agent, it is not limited thereto.

The fluorescence detection unit 12 and the return excitation light detection unit 13 are constituted of a well-known linear image sensor and detect the fluorescence of the PDT agent made incident from the optical system 20 and the return excitation light from the laser catheter 30, respectively. The fluorescence detection unit 12 and the return excitation light detection unit 13 supply the detected fluorescence and return excitation light to the control unit 14 as electric signals.

In this manner, information on not only the fluorescence but also the return excitation light is obtained, thus such a configuration can be used for monitoring an operating situation of the treatment device 1 to check whether the excitation light for the therapy is properly irradiated under predetermined conditions of a light quantity, etc.

The fluorescence detection unit 12 comprises a detection element such as a silicon photodiode, an avalanche photodiode, a spectrometer, and a photomultiplier tube. A measuring sampling rate of the detection element is 10 to 1000Hz and preferably 100 to 500Hz.

The control unit 14 controls the each unit in the treatment device 1.

The control unit 14 calculates fluorescence intensity and return excitation light intensity based on the electric signals obtained from the fluorescence detection unit 12 and the return excitation light detection unit 13, and then calculates information on a degree of ablation in real time based on these intensities.

The control unit 14 also outputs a display command to the display unit 16 for displaying calculation results.

The memory unit 17 is a non-volatile memory and set, for example, in a flash memory, an HDD, and other solid memories. The control unit 14 associates the calculated fluorescence intensity and excitation light intensity, information on a degree of ablation, and time information obtained from a time measuring unit not illustrated for measuring, for example, a passage time counted from a reference time such as an excitation light irradiation start time, with each other into the context and stores them in the memory unit 17 as a temporal change.

The display unit 16 is a display device, which uses, for example, a liquid-crystal display device and the like. Upon obtaining a display command from the control unit 14, the display unit 16 displays, for example, the information on a degree of ablation, the time information, and the like in real time on a display screen based on display information included in the display command.

The operation unit 15 accepts a command by an input operation from a practitioner and outputs the accepted command to the control unit 14. Examples of such a command include a command to turn on/off the excitation light output from the light source 11 and a command to change the intensity thereof.

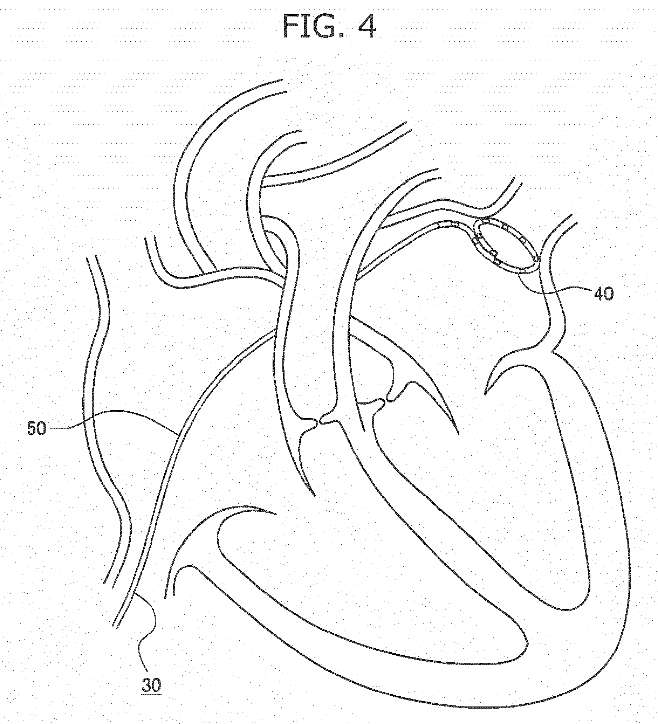

In the treatment device 1, the laser catheter 30 shown in Fig. 4 to Fig. 9 is detachably connected via a connector not illustrated to a tip of a tube, not illustrated, connected to the treatment device 1.

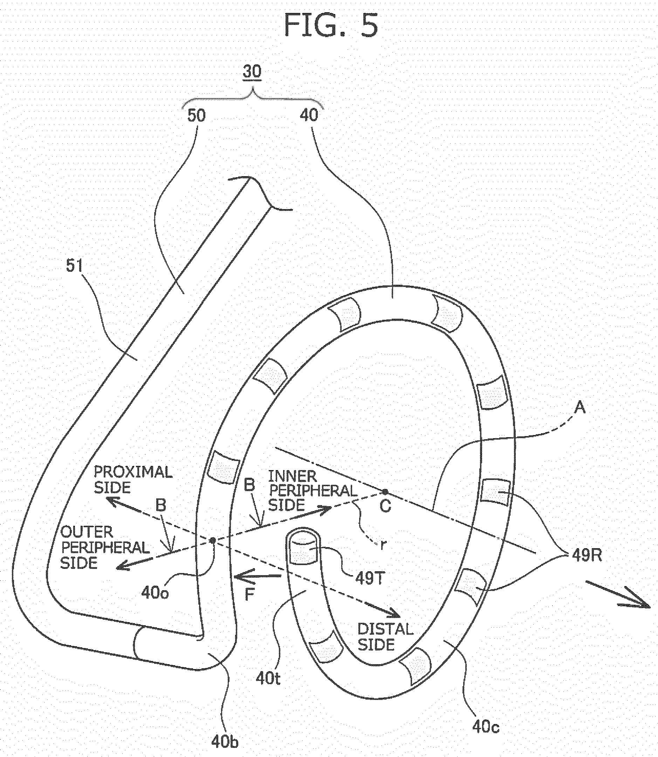

The laser catheter 30 comprises, as shown in Fig. 4 and Fig. 5, a head portion 40 arranged at the distal end of the laser catheter 30 and a lengthy tubular portion 50 connected to the head portion 40 at the proximal side of the head portion 40. A control handle not illustrated is connected to the proximal portion of the tubular portion 50 at the opposite side of the head portion 40. A connector not illustrated is arranged at the proximal portion of the control handle at the opposite side of the tubular portion 50.

<<Configuration of head portion 40>>

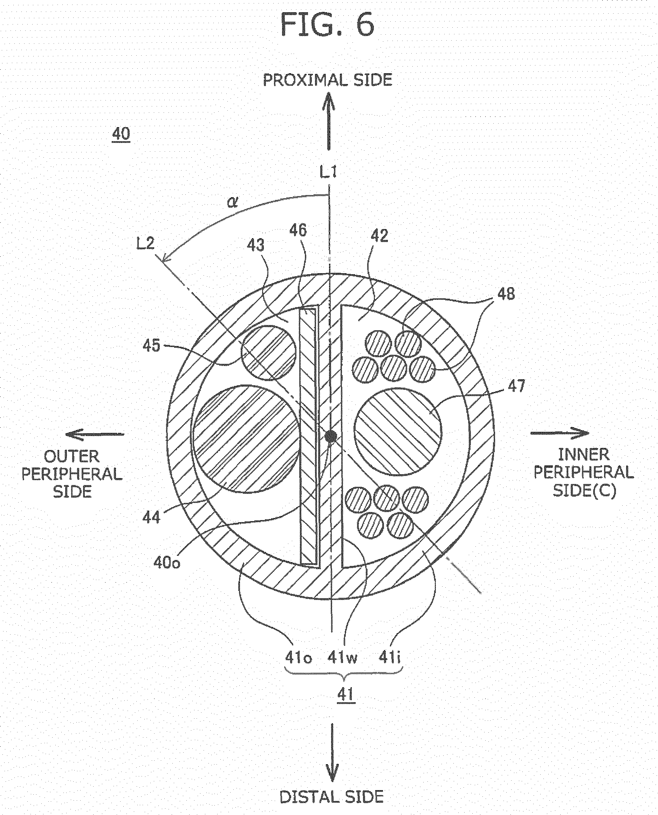

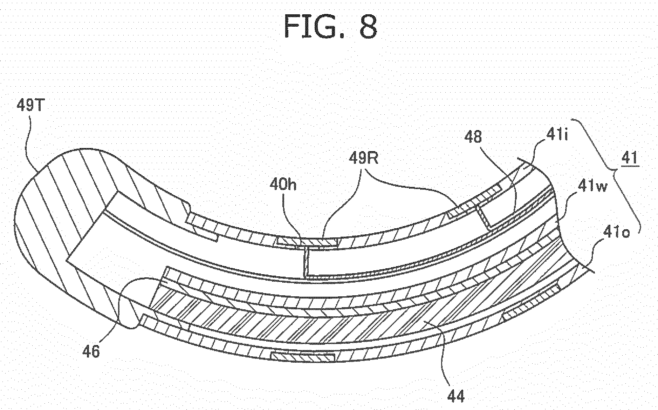

As shown in Fig. 6 and Fig. 8, the head portion 40 comprises a transparent tube 41 as a medical instrument body formed of a hollow flexible transparent material, in which the light diffuser for irradiation 44, the light diffuser for monitoring 45, a reflector 46, a shape memory wire 47, and lead wires 48 are inserted. The head portion 40 constitutes a loop portion, which is wound along over one circumference in a helical shape by the shape memory wire 47. A shape of the head portion 40 of the laser catheter 30 is also called as a lasso shape. The shape is not a closed annular shape to be precise, however it has a pseudo-ring structure, thus the shape is also called as a ring shape or an annular shape.

The transparent tube 41 is a soft hollow cylindrical transparent tube made of a polyether block amide copolymer (PEBAX (registered trademark), manufactured by Arkema) and the like. Other materials, which may be used for the transparent tube 41, are electrically nonconductive polymeric materials such as polyethylene terephthalate (PET), polytetrafluoroethylene (PTFE), fluorinated ethylene propylene (FEP), perfluoroalkoxy (PFA), urethane, and polyurethane. Alternatively, optically transparent and electrically nonconductive polymeric materials such as polyvinyl chloride (PVC) may be used.

As shown in Fig. 6 and Fig. 8, the transparent tube 41 comprises an inner peripheral side lumen 42 and an outer peripheral side lumen 43 partitioned by a partition wall 41 W.

It is noted that, in the present embodiment, the transparent tube 41 is constituted of an entirely transparent tube, however, it is not limited thereto, and only a wall portion 41o located at the outer peripheral side of the outer peripheral side lumen 43 needs to be transparent. Accordingly, a wall portion 41i located at the inner peripheral side and the partition wall 41W may be constituted of a semi-transparent or opaque material.

The inner peripheral side lumen 42 having an approximately semicircular cross section is located at the inner peripheral side, that is, the center C side of the helical shape, and partitioned by the wall portion 41i located at the inner peripheral side of the helical shape and the partition wall 41W of the transparent tube 41. In the inside of the inner peripheral side lumen 42, the shape memory wire 47 is inserted in the middle of the cross section in the longitudinal direction so as to face the wall portion 41i and the partition wall 41 W, and ten lead wires 48 are inserted on both end sides of the cross section in the longitudinal direction so as to sandwich the shape memory wire 47.

The shape memory wire 47 is formed of nickel-titanium alloy having an approximately semicircular cross section and exhibits a shape memory characteristic. For the shape memory wire 47, a ferrous shape memory alloy such as an iron-manganese-silicon-based alloy, a bimetal formed by sticking two metal plates having different thermal expansion coefficient, and the like may be used.

The shape memory wire 47 is formed so as to maintain a shape that a curving end of the helical shape that is wound around by about 1.1 circumferences is bent at a angle larger than or equal to a right angle, so that the shape of the head portion 40 shown in Fig. 4 and Fig. 5 is maintained when an external force is not applied thereto.

In Fig. 8, the shape memory wire 47 is not illustrated for the purpose of facilitating understanding of the drawing, however, the distal end portion of the shape memory wire 47 is fixed to a tip electrode 49T, while the proximal end portion is fixed to the tubular portion 50.

The shape memory wire 47 has elasticity and thus is deformable according to an external pressure applied thereto from the outside. Therefore, when the head portion 40 is placed inside the blood vessel, the head portion 40 is formed into a gentle curve shape and the like following the shape of the blood vessel by a pressure from a sidewall of the blood vessel.

The lead wires 48 are lead wires used in a conventional electrode catheter and each of the 10 lead wires 48 is correspondingly caulked and connected with nine ring-shaped electrodes 49R formed on the outer periphery of the head portion 40 and one tip electrode 49T by a well-known method.

The outer peripheral side lumen 43 having an approximately semicircular cross section is located at the outer peripheral side, that is, the opposite side of the center C of the helical shape, and partitioned by the wall portion 41o located at the outer peripheral side of the helical shape and the partition wall 41W of the transparent tube 41. The reflector 46 is arranged so as to abut on the partition wall 41 W inside of the outer peripheral side lumen 43. Further, the light diffuser for irradiation 44 is inserted in the middle of the cross section in the longitudinal direction so as to face the wall portion 41o and the reflector 46, and the light diffuser for monitoring 45 is inserted adjacent to the light diffuser for irradiation 44.

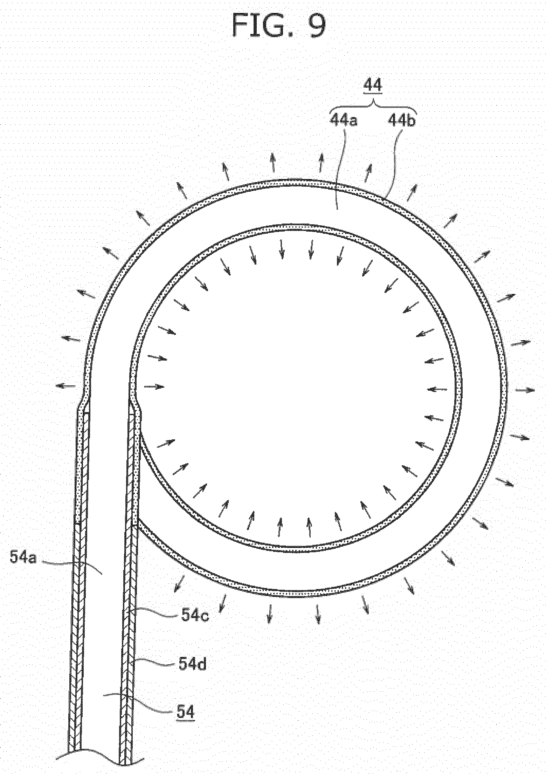

As shown in Fig. 9, the light diffuser for irradiation 44 is composed of a light diffuser body 44a formed by exposing a core, over a predetermine length from the tip portion 44t, of an optical fiber cable 54 inserted inside the tubular portion 50 by removing a clad 54C and a coating 54d, and a resin layer 44b which covers the outer periphery of the light diffuser body 44a.

The light diffuser body 44a is formed integrally with a core 54a of the optical fiber cable 54 inserted inside the tubular portion 50 and formed by a sand blasting process, so that emitted light to a lateral direction having an angle with respect to the longitudinal direction of the light diffuser body 44a is uniformized. It is noted that the light diffuser body 44a is not limited thereto, and it may comprise a hollow portion in the center thereof, and arrange a light reflection mirror or make indentations on its inner surface in order to uniformize light emitted to the lateral direction.

The resin layer 44b is formed, for example, by applying an acrylic ultraviolet curing resin in which a fine powder of quartz is dispersed and curing it by an ultraviolet ray.

An end portion of the optical fiber cable 54 located on the opposite side of the light diffuser for irradiation 44 is fixed to a connector not illustrated, and, by this connector, the optical fiber cable 54 is constituted so as to be connectable to a laser generating device not illustrated containing a laser source not illustrated.

It is noted that the light diffuser for irradiation 44 is not limited to a system shown in Fig. 9, and can employ other systems.

Systems constituting the light diffuser for irradiation 44 are roughly classified into two, namely, a system in which the light diffuser for irradiation 44 is constituted by extending the core 54a of the optical fiber cable 54 and s system in which the light diffuser for irradiation 44 is arranged as a different body from the core 54a. Either system can be used for the light diffuser for irradiation 44 of the present embodiment.

In the former, there is a case where the core 54a itself constitutes a diffusion substance and a case in which the core 54a does not. Specifically, the former is roughly classified into a transmitted light leaking system (e.g., a system in which the core 54a is partially exposed by causing fine flaws on the clad 54c and a system in which leaking is caused by bending) and a system in which a diffusion substance is used.

Examples of the transmitted light leaking system include a flaw processing (e.g., a sand blasting, a stamping, and a solvent treatment), a Fiber Bragg Grating (FBG) treatment, and a micro-bending treatment.

Further, examples of the system in which a diffusion substance is used include a system in which a diffusion substance is included in the core 54a/clad54c, and a system in which a diffusion substance is included in the coating 54d with the core 54a being exposed. It is noted that the sand blasting treatment is a method for blowing fine particles and thus can also fall in the system in which a diffusion substance is used.

The latter case in which the light diffuser for irradiation 44 is arranged as a different body from the core 54a includes a case in which an optical element different from the core 54a is used as the light diffuser for irradiation 44. In such a case, for example, an optical element such as a polyhedral prism and a SELFOC (registered trademark) lens (a refractive index distribution type lens) is used as the light diffuser for irradiation 44.

The light diffuser for irradiation 44 is extended over the entire length of the head portion 40, and a proper diameter of the helical shape in Fig. 5 is, although depending on a shape of the treatment target tissue, 5 to 50mm, preferably 5 to 15mm in case of being used for the photodynamic therapy for arrhythmia. Thus, the entire length of the light diffuser for irradiation 44 is 1.5 to 15cm, preferably about 3 to 9cm.

A diameter of the light diffuser for irradiation 44 is 0.1 to 1.0mm, preferably 0.25 to 0.75mm.

The light diffuser for irradiation 44 is used for irradiating light from the light source to the treatment target site.

A diameter of the light diffuser for monitoring 45 is 0.1 to 1.0mm, preferably 0.25 to 0.75mm.

The light diffuser for monitoring 45 has a similar configuration to the light diffuser for irradiation 44.

The light diffuser for monitoring 45 is used for receiving return fluorescence and measuring information on a degree of ablation.

The light diffuser for irradiation 44 and the light diffuser for monitoring 45 are adjacent to each other, thus, when both diffusers contact and rub against each other, there is concern that signal deterioration occurs due to a cross talk of signals that are leaked from the each diffuser.

As a measure to this problem, the light diffuser for irradiation 44 and the light diffuser for monitoring 45 may be adhered to each other by a transparent adhesive or a transparent spacer may be disposed between them.

It is noted that, in the present embodiment, the light diffuser for irradiation 44 and the light diffuser for monitoring 45 are constituted as different bodies, however, they may be constituted as an integrated body. Specifically, the light diffuser for irradiation 44 and the light diffuser for monitoring 45 may be constituted as a single light diffuser. When a single light diffuser is used, irradiation and light reception may be alternately performed on the time base by switching a time zone for irradiation and a time zone for light reception.

The reflector 46 is used to suppress radiation of light, irradiated from the light diffuser for monitoring 45, toward the inner peripheral side of the helical shape and facilitate the light radiation toward the outer peripheral side. By suppressing radiation toward the inner peripheral side of the helical shape, it becomes possible to focus light irradiation on the treatment target tissue and prevent light from being irradiated to a site other than the treatment target tissue. As a result, an amount of irradiation to the blood mostly present in the inner peripheral side is reduced and damages on the blood such as hemolysis can be suppressed.

The reflector 46 is made of an aluminum having a thickness of 0.01 to 0.10mm, preferably 0.03 to 0.08mm, and formed in a lengthy rectangular plate body having the same length as the light diffuser for irradiation 44 and the light diffuser for monitoring 45. The reflector 46 may be made of other materials having high reflectivity against excitation light, such as gold and silver. Further, instead of arranging the reflector 46, a plate that is curved into a U-shaped cross section or bent into a V-shaped cross section so as to position the center of the plate in the width direction on the center C side of the helical shape may be used. Alternatively, surfaces of the light diffuser for irradiation 44 and the light diffuser for monitoring 45 on the inner peripheral side of the helical shape may be coated with aluminum, gold, silver, and the like.

As shown in Fig. 5, the nine ring-shaped electrodes 49R and the one tip electrode 49T are arranged on the outer periphery of the head portion 40.

The ring-shaped electrodes 49R and the tip electrode 49T are constituted of a conductive material such as platinum, gold, iridium, or their alloys. The ring-shaped electrodes 49R are configured that the width of the electrodes in the extension direction of the head portion 40 is 1 mm or less, preferably 0.6mm or less so as not to shield light radiation. Further, inner surfaces of the ring-shaped electrodes 49R are preferably coated with gold in order to suppress light absorption by the ring-shaped electrodes 49R. Further, when the ring-shaped electrodes 49R are formed of gold, the light absorption by the ring-shaped electrodes 49R is suppressed, thus such a configuration is preferable.

As shown in Fig. 8, side holes 40h are formed in the head portion 40 at positions where the ring-shaped electrodes 49R are mounted, and through the side holes 40h, each of the nine lead wires 48 is connected to one of the corresponding ring-shaped electrodes 49R. Further, a tip hole is also formed at the distal tip of the head portion 40 and one of the lead wires 48 is connected to the tip electrode 49T through the tip hole.

The lead wire 48 is constituted by coating a metallic core wire having a diameter of about 0.075mm with a resin such as a polyamideimide resin having a film thickness of about 10μm.

Further, in the present embodiment, the nine ring-shaped electrodes 49R and the one tip electrode 49T are provided, however, the number of the ring-shaped electrodes 49R is not limited thereto, and it may be around 7 to 9. Further, when a diameter of a loop portion 40c is as small as approximately 5mm, the number of the ring-shaped electrodes 49R may be around 4.

The lead wires 48 are connected to a potential detection device not illustrated via a connector not illustrated. In this manner, potential signals derived in the ring-shaped electrodes 49R and the tip electrode 49T are inputted to the potential detection device not illustrated, and information on a contact state of each of the ring-shaped electrodes 49R and the tip electrode 49T with the treatment target tissue and information on the treatment depth are calculated by a well-known method and displayed.

By including the shape memory wire 47, the head portion 40 forms the following shape as shown in Fig. 5 when the external force is not applied thereto: the loop portion 40c is formed into the helical shape turned by about 1.1 circumferences at the distal tip of the head portion 40, and, at the proximal end of the loop portion 40c, the head portion 40 is formed into the bent shape at a bent portion 40b that is bent at a angle of about 90°C to 105°.

In the head portion 40, when a force F slightly pressing a distal tip portion 40t of the loop portion 40c along the direction of the center axis A of the loop portion 40c toward the bent portion 40b is applied, the tip portion 40t is brought into a contact with the bent portion 40b, and the head portion 40 forms a closed annular shape.

Therefore, just by slightly pressing the head portion 40 against an annular treatment target site such as a vein, as shown in Fig. 4, the head portion 40 forms a closed annular shape and also the light diffuser for irradiation 44 stored inside forms a closed annular shape, thus it becomes possible to form an abnormal electrical conduction blocking line without any gap.

It is noted that the head portion 40 may be formed into an annular shape in which the tip portion 40t and the bent portion 40b contact each other when the external force is not applied to the head portion 40.

In the example in Fig. 6, the partition wall 41 W and the reflector 46 are formed along a straight line L1 parallel to the center axis A of the loop portion 40c in Fig. 5. That is, the straight line L1 is perpendicular to a straight line r connecting the center 40o of the cross section of the head portion 40 and the center C of the loop portion 40c.

However, the partition wall 41W and the reflector 46 do not need to be formed along the straight line L1 and the partition wall 41 W and the reflector 46 may be formed along a straight line L2 shown in Fig. 6, which is inclined with respect to the straight line L1 at an angle of $\alpha°$ that satisfies $0°\leq \alpha \leq 90°$.

Fig. 6 shows a case of $\alpha$ =0°. In this case, the transparent tube 41 is separated into the inner peripheral side and the outer peripheral side with a plane parallel to the center axis A by the partition wall 41 W and the reflector 46. Accordingly, light is irradiated from the light diffuser for irradiation 44 toward the outer peripheral side in a direction substantially perpendicular to the straight line L1. Thus, in the case of $\alpha$=0° in Fig. 6, the head portion 40 is, for example, suitably applicable to a case in which the entire head portion 40 is inserted into a blood vessel that is slightly thicker than the head portion 40 and light is irradiated from the head portion 40 toward the outer peripheral side.

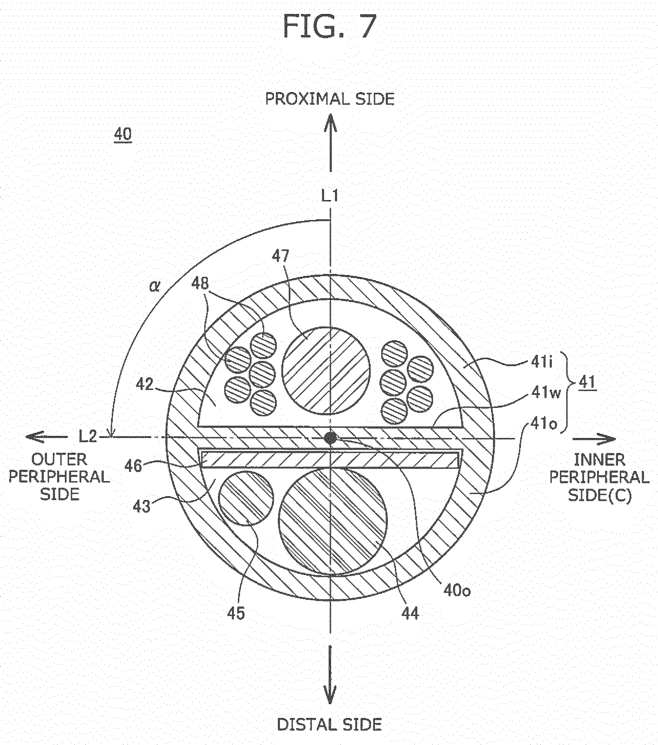

Further, Fig. 7 shows a case of $\alpha$=90°. In this case, the transparent tube 41 is separated into the proximal side and the distal side with a plane perpendicular to the center axis A by the partition wall 41W and the reflector 46. Accordingly, light is irradiated from the light diffuser for irradiation 44 toward the distal side in a direction along the straight line L1. Thus, in the case of $\alpha$=90° in Fig. 7, the head portion 40 is, for example, suitably applicable to a case in which the distal portion in the small helical shape having a diameter of about 5mm of the head portion 50 is pressed onto the distal treatment target site and irradiation is performed on a narrow region having a spot shape.

Further, the partition wall 41W and the reflector 46 may be arranged along the L2 in Fig. 2 inclined with respect to the L1 at an angle of $\alpha°$ that satisfies $0°<\alpha<90°$. In this case, the transparent tube 41 is separated into the proximal/inner peripheral side and the distal/outer peripheral side with a plane having an angle of more than 0° and less than 90° with respect to the center axis A by the partition wall 41W and the reflector 46. In this configuration, when compared with the case in which the partition wall 41W and the reflector 46 are arranged along the L2 in Fig. 6, an end portion at a treatment target tissue side (a distal end portion) of the partition wall 41W is inclined with respect to the straight line L1 so as to be positioned at the inner peripheral side of the helical shape. For example, when the loop portion 40c is pressed toward an inner surface of the pulmonary vein from the left atrium side, a treatment target tissue is positioned not exactly at the outer peripheral side of the loop portion 40c, but at a position slightly shifting to the distal side from the outer peripheral side. Thus, by directing the partition wall 41W and the reflector 46 to the distal/outer peripheral side, it becomes possible to perform light irradiation and measurement more efficiently.

The tubular portion 50 comprises the tube 51 constituting an outer hull thereof, in which the optical fiber cable 54 continuing to the light diffuser for irradiation 44, an optical fiber cable not illustrated continuing to the light diffuser for monitoring 45, and the ten lead wires 48 are inserted.

The tube 51 is formed by a resin, such as a polyether block amide copolymer (PEBAX (registered trademark), manufactured by Arkema), and electrically nonconductive polymeric materials such as polyethylene terephthalate (PET), polytetrafluoroethylene (PTFE), fluorinated ethylene propylene (FEP), perfluoroalkoxy (PFA), urethane, and polyurethane, or polyvinyl chloride (PVC). It is noted that the tube 51 does not need to be transparent.

The tube 51 has some flexibility (bendability), compression resistance in the tube axis direction, and torsional rigidity. By virtue of torsional rigidity of the tube 51, a rotation torque from the control handle not illustrated can be transmitted to the head portion 40.

The optical fiber cable 54, the optical fiber cable, not illustrated, continuing to the light diffuser for monitoring 45, and the ten lead wires 48 are extended to a connector not illustrated via the control handle, not illustrated, connected to the proximal portion of the tubular portion 50, and connected to a tip of a tube, not illustrated, connected to the treatment device 1 by the connector not illustrated.

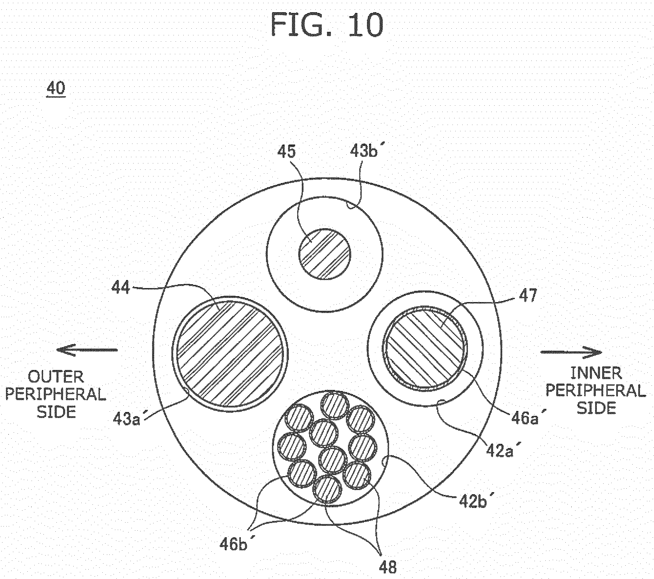

It is noted that a head portion 40' shown in Fig. 10 may be used for the laser catheter 30 instead of the head portion 40 shown in Fig. 6 and Fig. 8.

The head portion 40' comprises four lumens as a total, which are extended along the longitudinal direction of the head 40', as shown in Fig. 10.

Specifically, the head portion 40' comprises a wire lumen 42a' into which the shape memory wire 47 is inserted, a lead wire lumen 42b', into which the lead wires 48 are inserted, a light diffuser for irradiation lumen 43a', into which the light diffuser for irradiation 44 is inserted, and a light diffuser for monitoring lumen 43b', into which the light diffuser for monitoring 45 is inserted.

It is noted that when one light diffuser composed of a single body is arranged instead of the light diffuser for irradiation 44 and the light diffuser for monitoring 45, one lumen is enough for the light diffuser, thus the head portion 40' may be configured to have three lumens as a total.

Reflection coils 46a' and 46b' consisting of a rectangular wire (vertical) coil made of a material having high reflectivity to excitation light, including metal such as gold, silver, and aluminum, are wound around the shape memory wire 47 and the lead wires 48 over the entire length of the head portion 40'.

The reflection coils 46a' and 46b' perform a role as a reflector. By constituting the reflection coils 46a' and 46b' by the rectangular wire (vertical) coil, gaps formed between wire materials of the coils are eliminated and occupancy thereof is increased, thereby forming a reflector having an approximately cylindrical shape.

It is noted that, instead of arranging the reflection coils 46', the reflector may be constituted by coating inner peripheral surfaces of the wire lumen 42a' and the lead wire lumen 42b' with aluminum, gold, silver, and the like. Further, the reflector may be constituted by coating surfaces of the light diffuser for irradiation 44 and the light diffuser for monitoring 45 on the inner peripheral side in the helical shape with aluminum, gold, silver, and the like.

The description of other configurations of the head portion 40' is omitted as they are the same as the head portion 40.

«Regarding treatment and measurement of treatment depth using laser catheter 30»

Next, explanation is given to procedures for performing the photodynamic therapy for arrhythmia as well as measuring information on a degree of ablation in the photodynamic therapy by the treatment device 1 using the laser catheter 30 of the present embodiment.

First, the laser catheter 30 is inserted in the heart via the femoral vein or the jugular vein, not illustrated, of a patient by a practitioner such as a medical doctor. A tip portion of the laser catheter 30 is placed in an inner wall of the cardiac-muscle tissue of the left atrium in the vicinity of the pulmonary vein.

Subsequently, a PDT agent of an amount necessary for the therapy is administered to the patient at once by intravenous injection by the practitioner. The administered PDT agent is diffused in the interstitium or the blood vessel.

After administering the PDT agent, a switch of the light source 11 not illustrated is turned on by the practitioner after a predetermined period of time, for example, several minutes to several tens of minutes, to initiate emission of excitation light from the light source 11. Then, the excitation light is irradiated from the entire length of the light diffuser for irradiation 44 of the laser catheter 30.

When the switch of the light source 11 is turned on at the light irradiation start time, a timer not illustrated is turned on.

The control unit 14 calculates fluorescence intensity detected by the fluorescence detection unit 12 at a time point when the switch of the light source 11 is turned on and stores this in a memory not illustrated.

Next, the control unit 14 determines whether a predetermined measurement time interval $\Delta t$ (sec.) (e.g., about 0.05 sec. to 0.5 sec.) has elapsed from a previous calculation time point of fluorescence intensity. If the $\Delta t$ (sec.) has elapsed, fluorescence intensity at that time point, detected by the fluorescence detection unit 12, is calculated and stored in the memory not illustrated.

The control unit 14 obtains a light irradiation time indicating a time from the light irradiation start time up to that time point from the timer not illustrated, and calculates return excitation light intensity at that time point detected by the return excitation light detection unit 13 at that point, and then, by using the fluorescence intensity calculated at that time point, the control unit 14 calculates an estimated treatment depth $d_{nec}$ according to the previous formula I (2).

Next, the control unit 14 sends a data of the estimated treatment depth $d_{nec}$ that is calculated to the display unit 16 and also stores the data in the memory not illustrated.

With this process, the estimated treatment depth $d_{nec}$ is displayed in the display unit 16 in real time for allowing visual inspection by the practitioner.

In this process, the estimated treatment depth $d_{nec}$ is displayed not as an absolute depth of ablation caused to the tissue by the photodynamic therapy, but as an indicator value thereof expressed as a relative value. Examples of such an indicator value include an integer or a number having the first or second decimal digit, such as "3" and "5.3".

It is noted that, instead of the estimated treatment depth $d_{nec}$, a corrected estimated treatment depth $d_{nec}$ may be calculated using the corrected PBI corrected by the correction methods that utilize physiological information on a tissue and the like obtained by other methods, and displayed and stored.

Further, in the present embodiment, the PBI or the corrected PBI is used as an indicator value of information on the degree of ablation in the photodynamic therapy, however it is not limited thereto, and the fluorescence intensity detected by the fluorescence detection unit 12 may be used as information on the degree of ablation. In this case, the fluorescence intensity is, as it is or after being suitably processed, displayed in the display unit 16 and stored in the memory not illustrated.

Subsequently, the control unit 14 determines whether the predetermined measurement time elapses or whether the light source 11 is switched off, and if the measurement time does not elapse and the light source 11 is not switched off, the control unit 14 repeats calculation and display of the estimated treatment depth $d_{nec}$.

If the predetermined measurement time elapses or the light source 11 is switched off, it is interpreted that the photodynamic therapy is ended and the control unit 14 stores the data stored in the memory not illustrated in a memory unit 17, thereby completing the process.

EXAMPLES

Hereinafter, the present invention is further specifically described by showing specific Examples.

<<Verification test 1: fluorescence measurement test using light diffuser for irradiation and light diffuser for monitoring>>

In the present test, it was verified by an ex vivo test whether fluorescence of a PDT agent can be measured when the light diffuser for irradiation 44 and the light diffuser for monitoring 45 are arranged adjacent to each other.

The present test was performed by the following procedures.

A swine cardiac muscle tissue cut out in a thickness of 15 to 20mm was immersed in a talaporfin sodium ($30\mu g/ml$) solution as a PDT agent for 2 hours to prepare Example 1, while the swine cardiac muscle tissue cut out in the same manner, but not being immersed in the agent, was prepared as Comparative example 1.

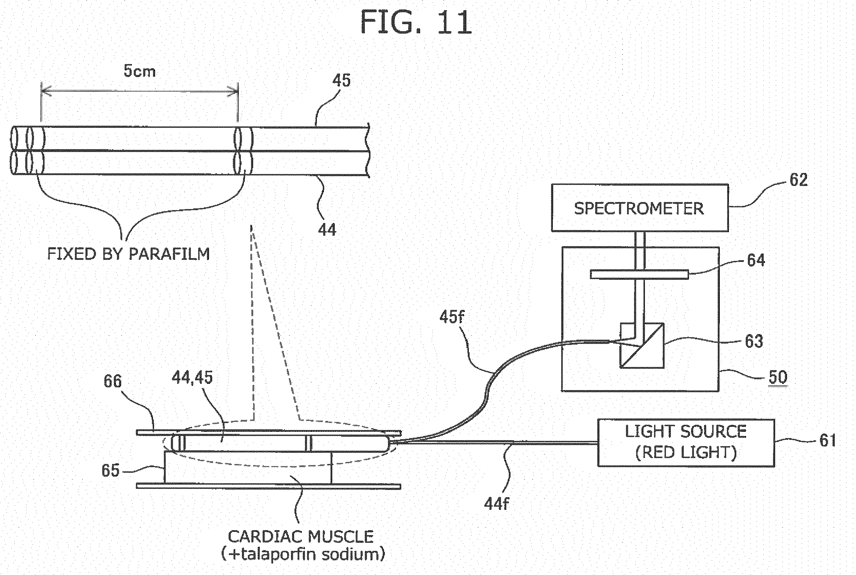

A device configuration of a fluorescence measuring system in Example 1 and Comparative example 1 is shown in Fig. 11. The optical fibers 44f and 45f composed of two diffusion-type plastic optical fibers of $500\mu m$, NA 0.5 and a diffusion length of 5cm (Cylindrical probe, manufactured by Medlight SA) were placed in parallel and sealed to each other by Parafilm, and then fixed on a cardiac muscle tissue 65 of Example 1 in a manner that the entire length of the diffuser portion located at the tip was brought into a contact with the cardiac muscle tissue 65. Between the two diffusion-type plastic optical fibers, one diffusion portion of the optical fiber 44f was designated as the light diffuser for irradiation 44, while the other diffusion portion of the optical fiber 45f was designated as the light diffuser for monitoring 45. A glass plate 66 was placed on the cardiac muscle tissue, the light diffuser for irradiation 44, and the light diffuser for monitoring 45.

A tip of the optical fiber 44f was connected to a light source 61 of red laser (Rouge-LD-02B, manufactured by Cyber Laser Inc., $663\pm2nm$), while a tip of the optical fiber 45f was connected to a dichroic mirror 63 of an optical system 60.

The optical system 60 comprises the dichroic mirror 63 (XF2024, manufactured by Omega Optical, Inc.), and a long-pass filter 64 (LVX0690, manufactured by Asahi Spectra Co., Ltd.), and is connected to a spectrometer 62 (PMA12, manufactured by Hamamatsu Photonics K.K.).

In the configuration in Fig. 11, a laser light having a wavelength of 663±2nm was light-irradiated from the optical fiber 44f via the light diffuser for irradiation 44 with an input power of 0.3W for 200 seconds. During the light irradiation, return light received from the optical fiber 45f via the light diffuser for monitoring 45 was measured for spectrum over time using the spectrometer 62 under conditions of a wavelength of 300 to 1050nm and an integration time of 500ms.

Further, the same measurement was performed (Comparative example 1) with the swine cardiac muscle tissue of Comparative example 1, which was not immersed in the agent. In addition, spectrum was measured over time using the swine cardiac muscle tissue of Comparative example 1, which was not immersed in the agent, under the same conditions, but without performing light irradiation by the light diffuser for irradiation 44 via the optical fiber 44f (Control 1).

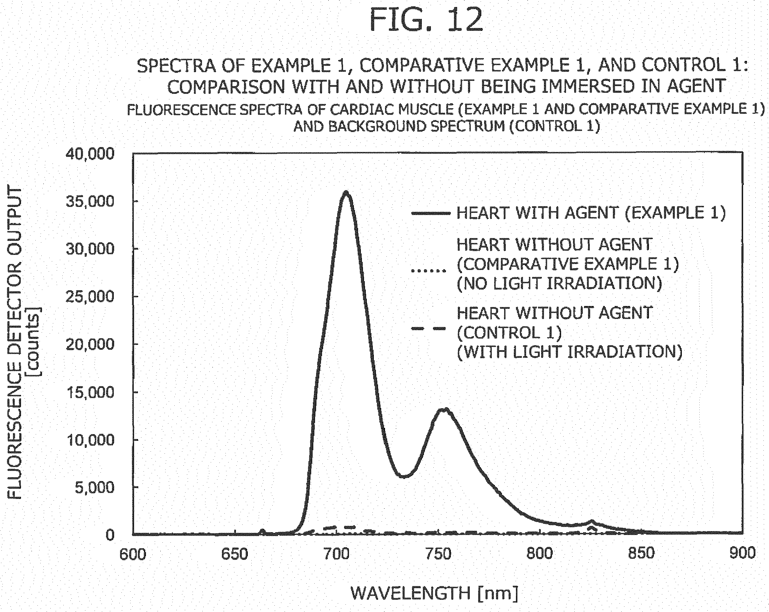

Spectra of Example 1, Comparative example 1, and Control 1 are shown in Fig. 12.

Fig. 12 showed that the return fluorescence from the light diffuser for monitoring 45 was detected only in Example 1 in which the cardiac muscle tissue was immersed in the agent, demonstrating that the return fluorescence intensity of the treatment target tissue could be measured when the light diffuser for irradiation 44 and the light diffuser for monitoring 45 were arranged adjacent to each other so as to face the treatment target tissue as shown in Fig. 12 in the presence of the PDT agent.

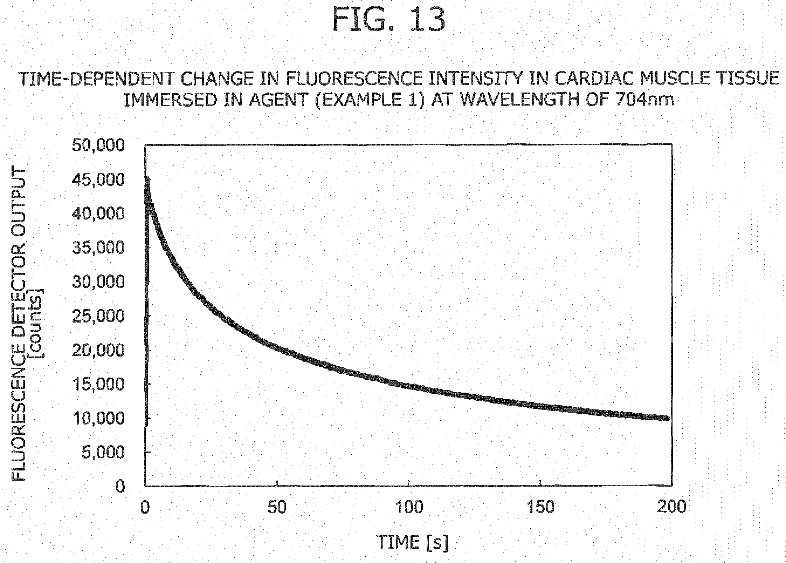

A change of fluorescence intensity over time measured at a wavelength of 704nm in Example 1 is shown in Fig. 13. From the result in Fig. 12, the fluorescence intensity became maximum at a wavelength of 704nm, thus a change of the fluorescence intensity at a wavelength of 704nm was examined over time.

Fig. 13 showed that the return fluorescence intensity of the cardiac muscle tissue at a wavelength of 704nm in Example 1 was reduced to a level of approximately one fourth of that at the irradiation start time over 200 seconds and the time-dependent change of the return fluorescence intensity in the cardiac muscle tissue at a wavelength of 704nm in Example 1 was similar to the time-dependent change of the fluorescence intensity, shown in Fig. 2, characteristic to the extracellular photosensitive reaction. Accordingly, it was demonstrated that a time-dependent change curve required for calculating the estimated treatment depth $d_{nec}$ in the photodynamic therapy using the light diffuser for irradiation 44 could be obtained by the device configuration of the fluorescence measuring system in Fig. 11.

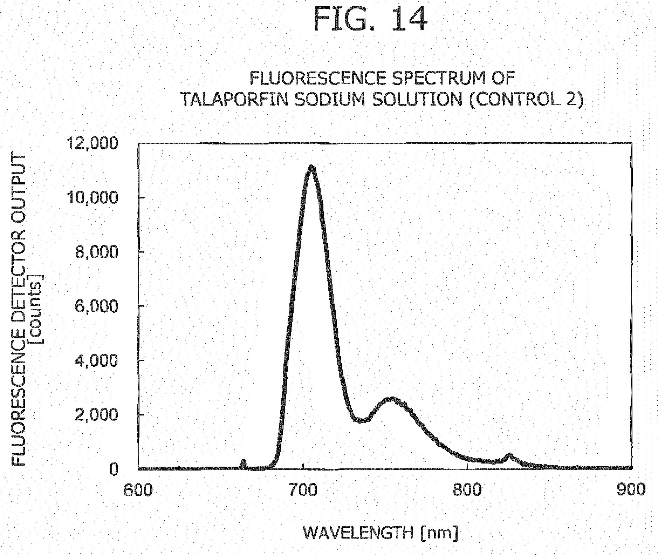

Further, Fig. 14 shows a fluorescence spectrum of return fluorescence measured on the same conditions using a solution containing physiological saline and talaporfin sodium (30µg/ml, no serum protein), instead of arranging the cardiac muscle tissue 65, with the device configuration of the fluorescence measuring system in Fig. 11 (Control 2). In Control 2, the fluorescence intensity was measured by directly placing the light diffuser for irradiation 44 and the light diffuser for monitoring 45 in the solution containing physiological saline and talaporfin sodium (30µg/ml) with the device configuration in Fig. 11.

As shown in Fig. 14, a shape of the fluorescence spectrum obtained in Control 2 is substantially matched with a shape of the spectrum in Fig. 12, thus it was found that the fluorescence spectrum obtained in Fig. 12 was from the fluorescence derived from the agent. Accordingly, it was demonstrated that a value of the return fluorescence intensity obtained by the system in which the light diffuser for irradiation 44 and the light diffuser for monitoring 45 were arranged adjacent to each other could be used for calculating the estimated treatment depth $d_{nec}$ in the photodynamic therapy.

« Verification test 2»

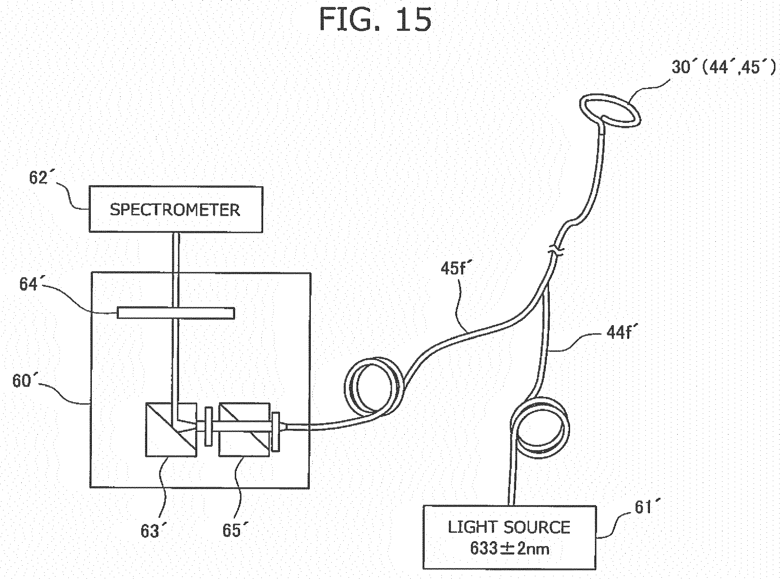

The present test was conducted to investigate whether monitoring of fluorescence could be performed by a loop-type laser catheter in an in vivo animal test under a catheter intervention using a device configuration of a fluorescence measuring system in Fig. 15. Two plastic optical fibers (diameter of 250µm) having a diffusion length of 7cm were externally mounted on a loop-shaped tip portion of a conventional general-purpose catheter having a distal tip portion formed in a loop shape to produce a laser catheter 30'. Verification test of the fluorescence monitoring by the laser catheter, as the medical instrument of the present invention, was performed by irradiating diffused excitation light from the one optical fiber and receiving fluorescence by the other optical fiber. Below, the content of the present verification test is explained.

The present test was conducted by the following procedures.

A sheath (10Fr.) was inserted and placed in the right femoral vein of a dog (hybrid, male, weight of 19kg). Next, the laser catheter 30' having a loop shape was inserted into the sheath and placed in the superior vena cava. In this test, the loop-type laser catheter 30' was produced by externally mounting two plastic optical fibers 44f' and 45f' (diameter of 250µm) having a diffusion length of 7cm on a general-purpose catheter, along an extension direction thereof, having a loop shape (a lasso type) and used for mapping, cauterization, or the like of the pulmonary vein. A light diffuser for irradiation 44' and a light diffuser for monitoring 45' having a diffusion length of 7cm are arranged at the tips of the two optical fibers 44f' and 45f' through a length of one circumference of the loop portion of the catheter.

A tip of the optical fiber 44f' was connected to a light source 61' of red laser (Rouge-LD-02B, manufactured by Cyber Laser Inc., 663±2nm), while a tip of the optical fiber 45f' was connected to a polarization beam splitter 65' of an optical system 60'.

The optical system 60 comprises the polarization beam splitter 65', a dichroic mirror 63' (XF2024, manufactured by Omega Optical, Inc.), and a long-pass filter 64' (LVX0690, manufactured by Asahi Spectra Co., Ltd.), and is connected to a spectrometer 62' (PMA12, manufactured by Hamamatsu Photonics K.K.).

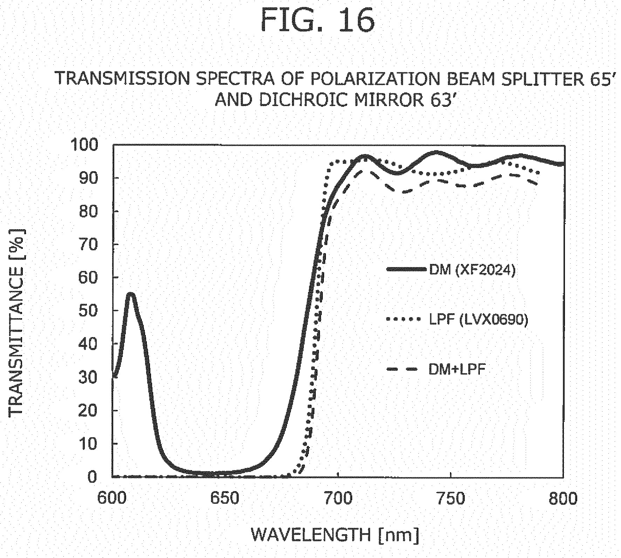

Fig. 16 in the drawings shows a filter characteristic at a wavelength of 600 to 790nm when the dichroic mirror 63' is used singly, the long-pass filter 64' is used singly, and the dichroic mirror 63' and the long-pass filter 64' are simultaneously used. In Fig. 16, DM refers to the dichroic mirror 63' and LPF refers to the long-pass filter 64'.

Next, a position of the catheter was adjusted under fluoroscopy so that the light diffuser for irradiation 44' and the light diffuser for monitoring 45' were brought into a contact with the superior vena cava, and talaporfin sodium (2.5mg/kg bolus, 2.93mg/kg/hr infusion) as a PDT agent was intravenously administered.

Sixty-two minutes after the administration of the agent (agent concentration in blood plasma: 42 to 48$\mu$g/ml), red light ($\lambda$=663±2nm) having an input power of 0.3W was guided into the light diffuser for irradiation 44' from the light source 61' and light irradiation on the superior vena cava was continuously performed for 3 minutes.

During light irradiation, return fluorescence from the light diffuser for monitoring 45' was measured over time using the spectrometer 62' under conditions of a wavelength of 650 to 900nm and an integration time of 500ms.

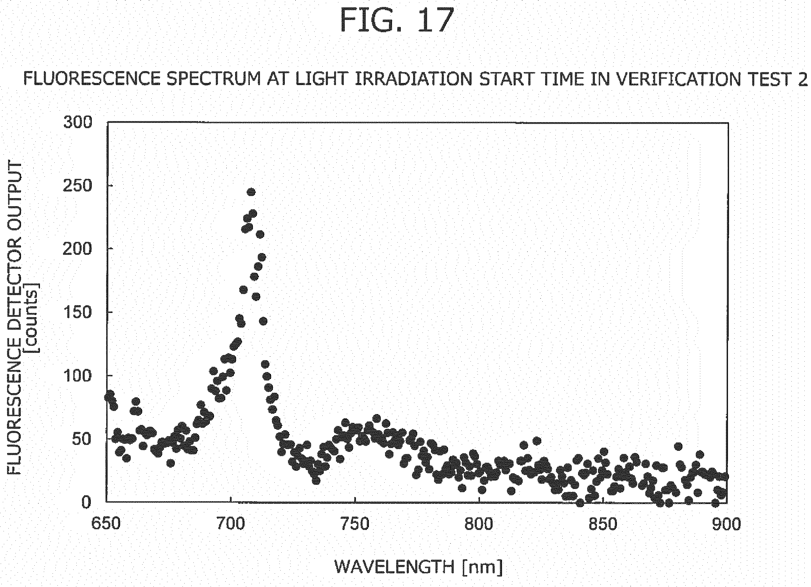

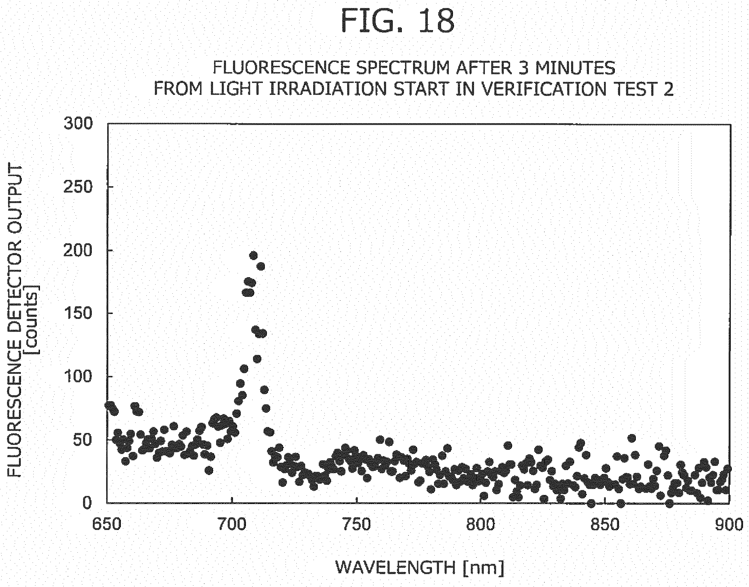

Measurement results are shown in Fig. 17 and Fig. 18. Fig. 17 is a graph showing detection values of the return fluorescence at a wavelength of 650 to 900nm at the light irradiation start time, and Fig. 18 is a graph showing detection values of the return fluorescence at a wavelength of 650 to 900nm after 3 minutes from the light irradiation start time.

From Fig. 17 and Fig. 18, focusing on fluorescence peak intensity derived from the agent at a wavelength of about 710nm, the fluorescence intensity was reduced 3 minutes after the start of irradiation as compared to that at the irradiation start time. This could confirm that the return fluorescence was attenuated as the light irradiation continued.

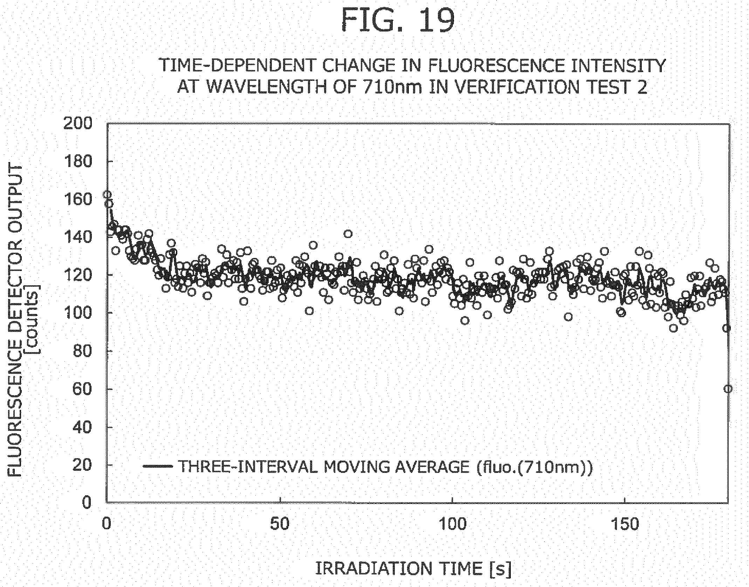

Further, a time-dependent change in the fluorescence intensity measured at a wavelength of 710nm in the present verification test is shown in Fig. 19. A change in fluorescence could be observed at a wavelength of 710nm without being influenced by excitation light ($\lambda$=663nm), thus a time-dependent change in the fluorescence intensity was verified at a wavelength of 710nm.

In the graph in Fig. 19, measurement data was plotted as an each dot and a three-interval moving average was shown with a solid line. In this graph, the moving average was obtained by calculating an average value within a specified interval while shifting the interval in a time series and thus can show a long-term changing trend on the graph.

In the graph in Fig. 19, the fluorescence intensity was reduced over time and a bleaching of fluorescence over time could be confirmed.

From the result in Fig. 19, it was demonstrated that the time-dependent change curve required for calculating the estimated treatment depth $d_{nec}$ in the photodynamic therapy using the light diffuser for irradiation 44' could be obtained by the device configuration of the fluorescence measuring system in Fig. 15.

REFERENCE SIGNS LIST

| A: | Center axis |
|---|---|
| C: | Center |
| F: | Force |
| L1, L2, r: | Straight lines |
| 1: | Treatment device |
| 11, 61, 61': | Light sources |
| 12: | Fluorescence detection unit |
| 13: | Return excitation light detection unit |
| 14: | Control unit |
| 15: | Operation unit |
| 16: | Display unit |
| 17: | Memory unit |
| 20, 60, 60': | Optical systems |
| 21, 65': | Polarization beam splitters |
| 22, 44f, 45f, 44f', 45f': | Optical fibers |
| 23, 63: | Dichroic mirrors |
| 24: | Band pass filter |
| 30, 30': | Laser catheters |
| 40, 40': | Head portions |

| 40c: | Loop portion |
|---|---|
| 40b: | Bent portion |
| 40h: | Side hole |
| 40o: | Center |
| 40t: | Tip portion |
| 41: | Transparent tube |
| 41i, 41o: | Wall portions |
| 41w: | Partition wall |
| 42: | Inner peripheral side lumen |
| 42a': | Wire lumen |
| 42b': | Lead wire lumen |
| 43: | Outer peripheral side lumen |
| 43a': | Light diffuser for irradiation lumen |
| 43b': | Light diffuser for monitoring lumen |
| 44, 44': | Light diffusers for irradiation |
| 44a: | Light diffuser body |
| 44b: | Resin layer |
| 44t: | Tip portion |
| 45, 45': | Light diffusers for monitoring |
| 46a', 46b': | Reflection coils |
| 46: | Reflector |
| 47: | Shape memory wire |
| 48: | Lead wire |
| 49T: | Tip electrode |
| 49R: | Ring-shaped electrode |
| 50: | Tubular portion |
| 51: | Tube |
| 54: | Optical fiber cable |
| 54c: | Clad |
| 54d: | Coating |
| 62, 62': | Spectrometers |
| 64, 64': | Long-pass filters |
| 65: | Cardiac muscle tissue |
| 66: | Glass plate |

**Claims**

1. A medical instrument that is inserted in a living body and irradiates light on a treatment target site for treating the site, comprising:

   a lengthy medical instrument body comprising at least one lumen; and, in the medical instrument body,
   a lengthy light diffuser and a lengthy reflector comprising a reflection surface for reflecting light, each being inserted to be extended along a longitudinal direction of the medical instrument body, wherein:

   the medical instrument body comprises, at least on a portion of an outer wall thereof, a transparent outer wall portion that is optically transparent and extended along the longitudinal direction; and
   the light diffuser comprises an irradiation section that irradiates light on the treatment target site and a light receiving section that receives light from the treatment target site and is arranged to face the reflection surface and the transparent outer wall portion in a position between the reflection surface and the transparent outer wall portion.

2. The medical instrument according to claim 1, wherein:

   the light diffuser comprises a lengthy light diffuser for irradiation constituting the irradiation section, extended along the longitudinal direction of the medical instrument body, and a lengthy light diffuser for receiving light constituting the light receiving section, formed as a different body from the light diffuser for irradiation and extended along the longitudinal direction of the medical instrument body; and

the light diffuser for irradiation and the light diffuser for receiving light are adjacently arranged so as to face the reflection surface and the transparent outer wall portion in a position between the reflection surface and the transparent outer wall portion.

3. The medical instrument according to claim 1 or 2, wherein:

the medical instrument comprises a tip portion composed of a loop portion that is wound along over one circumference in a helical shape when an external force is not applied thereto and a bent portion bent at an end portion of the loop portion at a proximal side of the medical instrument; and
the light diffuser for irradiation and the light diffuser for receiving light are extended in a region, of the tip portion, that is wound along over one circumference in the helical shape.

4. The medical instrument according to claim 3, wherein the reflection surface faces to an outer peripheral side in the helical shape and the light diffuser for irradiation and the light diffuser for receiving light are positioned at the outer peripheral side of the reflection surface.

5. The medical instrument according to claim 3 or 4, wherein a plurality of electrodes are arranged around the tip portion at predetermined intervals in an extension direction of the tip portion.

6. The medical instrument according to claim 5, wherein:

a shape memory member having shapes of the loop portion and the bent portion, when the external force is not applied thereto, is stored inside the tip portion; and
the medical instrument body comprises a plurality of lumens extended along an extension direction of the medical instrument body, wherein the light diffuser for irradiation and the light diffuser for receiving light, lead wires of the electrodes, and the shape memory member are stored in the different lumens.

7. The medical instrument according to claim 6, wherein the plurality of the lumens are divided into an inner peripheral side lumen arranged on an inner peripheral side of the helical shape and an outer peripheral side lumen arranged on an outer peripheral side, wherein:

in the inner peripheral side lumen, the shape memory member and a plurality of the lead wires connected to the plurality of the electrodes are stored; and
in the outer peripheral side lumen, the reflector is arranged adjacent to the inner peripheral side lumen, and the light diffuser for irradiation and the light diffuser for receiving light are arranged on the outer peripheral side of the reflector.

8. The medical instrument according to claim 6, wherein the plurality of the lumens are composed of:

a shape memory member lumen for storing the shape memory member;
a lead wire lumen for storing the plurality of the lead wires connected to the plurality of the electrodes;
a light diffuser for irradiation lumen for storing the light diffuser for irradiation; and
a light diffuser for receiving light lumen for storing the light diffuser for receiving light, arranged adjacent to the light diffuser for irradiation lumen in a circumferential direction of the medical instrument body, wherein the light diffuser for irradiation lumen and the light diffuser for receiving light lumen are arranged on the outer peripheral side of the medical instrument body.

9. The medical instrument according to claims 1 to 8, wherein the medical instrument is a laser catheter used for a photodynamic therapy for arrhythmia.

10. A light-ray treatment device, comprising:

the medical instrument according to claims 1 to 9;
a light source that projects excitation light to the light diffuser of the medical instrument;
a detection section that detects fluorescence received from the light diffuser of the medical instrument; and
an arithmetic section that receives signals of the detected excitation light and fluorescence from the detection section and calculates a progress of a light-ray treatment performed by the medical instrument based on the signals.

**Amended claims under Art. 19.1 PCT**

1. (Amended) A medical instrument that is inserted in a living body and irradiates light on a treatment target site for treating the site, comprising:

   a lengthy medical instrument body comprising at least one lumen; and, in the medical instrument body,
   a lengthy light diffuser and a lengthy reflector comprising a reflection surface for reflecting light, each being inserted to be extended along a longitudinal direction of the medical instrument body, wherein:

   the medical instrument body comprises, at least on a portion of an outer wall thereof, a transparent outer wall portion that is optically transparent and extended along the longitudinal direction;
   the light diffuser comprises an irradiation section that irradiates light on the treatment target site and a light receiving section that receives light from the treatment target site, and is arranged to face the reflection surface and the transparent outer wall portion in a position between the reflection surface and the transparent outer wall portion;
   the medical instrument further comprises a tip portion that is equipped with electrodes and comprises a loop portion that is wound along over one circumference in a helical shape when an external force is not applied thereto, and
   a bent portion that is bent at an end portion of the loop portion at a proximal side of the medical instrument;
   in an inside of the tip portion, a shape memory member having shapes of the loop portion and the bent portion, when the external force is not applied thereto, is stored;
   the medical instrument body comprises an inner peripheral side lumen arranged on an inner peripheral side of the helical shape and an outer peripheral side lumen arranged on the outer peripheral side, each being extended into an extension direction of the medical instrument body;
   in the inner peripheral side lumen, the shape memory member and lead wires connected to the electrodes are stored; and
   in the outer peripheral side lumen, the reflector is arranged adjacent to the inner peripheral side lumen and the light diffuser is arranged on the outer peripheral side of the reflector.

2. (Amended) The medical instrument according to claim 1, wherein:

   the light diffuser comprises a lengthy light diffuser for irradiation constituting the irradiation section, extended along the longitudinal direction of the medical instrument body, and a lengthy light diffuser for receiving light constituting the light receiving section, formed as a different body from the light diffuser for irradiation and extended along the longitudinal direction of the medical instrument body; and
   the light diffuser for irradiation and the light diffuser for receiving light are adjacently arranged.

3. (Amended) The medical instrument according to claim 2, wherein the light diffuser for irradiation and the light diffuser for receiving light are extended in a region, of the tip portion, that is wound along over one circumference in the helical shape.

4. (Amended) The medical instrument according to claim 2, wherein the reflection surface faces to an outer peripheral side in the helical shape and the light diffuser for irradiation and the light diffuser for receiving light are positioned at the outer peripheral side of the reflection surface.

5. (Amended) The medical instrument according to claim 1, wherein a plurality of the electrodes are arranged around the tip portion at predetermined intervals in an extension direction of the tip portion.

6. (Amended) The medical instrument according to claim 1, wherein:

   the inner peripheral side lumen comprises:

   a shape memory member lumen for storing the shape memory member; and
   a lead wire lumen for storing a plurality of the lead wires connected to the plurality of the electrodes;

   the outer peripheral side lumen comprises:

   a light diffuser for irradiation lumen for storing the light diffuser for irradiation; and

a light diffuser for receiving light lumen for storing the light diffuser for receiving light, arranged adjacent to the light diffuser for irradiation lumen in a circumferential direction of the medical instrument body; and

the light diffuser for irradiation lumen and the light diffuser for receiving light lumen are arranged on the outer peripheral side of the medical instrument body.

7. (Amended) The medical instrument according to claim 1, wherein the medical instrument is a laser catheter used for a photodynamic therapy for arrhythmia.

8. (Amended) A light-ray treatment device, comprising::

the medical instrument according to claim 1;
a light source that projects excitation light to the light diffuser of the medical instrument;
a detection section that detects fluorescence received from the light diffuser of the medical instrument; and
an arithmetic section that receives signals of the detected excitation light and fluorescence from the detection section and calculates a progress of a light-ray treatment performed by the medical instrument based on the signals.

9. (Canceled)

10. (Canceled)
Restriction of claim 1 is made as follows by adding the requirement described in claim 7 in the international application as filed to amended claim 1: "the medical instrument body comprises an inner peripheral side lumen arranged on an inner peripheral side of the helical shape and an outer peripheral side lumen arranged on the outer peripheral side, each being extended into an extension direction of the medical instrument body, wherein: in the inner peripheral side lumen, the shape memory member and lead wires connected to the electrodes are stored; and in the outer peripheral side lumen, the reflector is arranged adjacent to the inner peripheral side lumen and the light diffuser is arranged on the outer peripheral side of the reflector". This requirement is not described in the five cited literatures (JP2012-510084A, JP2008-520363A, JP2013-13726A, JP2009-56290A, and JP2011-212423A) assigned as Category "Y" in the International Search Report.
As a result of having the configuration according to amended claim 1, the present invention obtains the advantageous effect as follows: "the light diffuser is located on the outer peripheral side of the helical shape and other constituent elements are located on the inner peripheral side, thus irradiation of light toward the outside of the helical shape and reception of light from the outside of the helical shape can be ensured. Thus, it becomes possible to perform light irradiation that is focused on the treatment target site, and, in the same time, a site other than the treatment target site can be prevented from receiving light irradiation. As a result, the amount of irradiation to the blood that is not the treatment target is reduced and damages on the blood such as hemolysis can be suppressed." (paragraph [0022] of the description in the international application as filed).

# FIG. 1

FIG. 2

# FIG. 3

FLUORESCENCE INTENSITY
AT LIGHT IRRADIATION START TIME

FIRST LIGHT EMISSION PERIOD

SECOND LIGHT EMISSION PERIOD (STEADY STATE)

FLUORESCENCE DETECTOR OUTPUT [V]

IRRADIATION TIME[s]

RATIO BETWEEN FLUORESCENCE INTENSITY AT LIGHT IRRADIATION START TIME AND FLUORESCENCE INTENSITY AT STEADY STATE

TIME OF FIRST LIGHT EMISSION PERIOD

# FIG. 4

# FIG. 5

# FIG. 6

# FIG. 7

PROXIMAL SIDE

40

L1

α

47

48

41i
41w    41
41o

42

L2

OUTER
PERIPHERAL
SIDE

46

INNER
PERIPHERAL
SIDE(C)

43

40o

45    44

DISTAL SIDE

# FIG. 8

# FIG. 9

# FIG. 10

# FIG. 11

5cm

45

44

FIXED BY PARAFILM

66

44,45

65

CARDIAC MUSCLE
(+talaporfin sodium)

SPECTROMETER — 62

64

45f

63

50

LIGHT SOURCE
(RED LIGHT) — 61

44f

# FIG. 12

SPECTRA OF EXAMPLE 1, COMPARATIVE EXAMPLE 1, AND CONTROL 1:
COMPARISON WITH AND WITHOUT BEING IMMERSED IN AGENT

FLUORESCENCE SPECTRA OF CARDIAC MUSCLE (EXAMPLE 1 AND COMPARATIVE EXAMPLE 1)
AND BACKGROUND SPECTRUM (CONTROL 1)

Legend:
— HEART WITH AGENT (EXAMPLE 1)

...... HEART WITHOUT AGENT (COMPARATIVE EXAMPLE 1) (NO LIGHT IRRADIATION)

– – HEART WITHOUT AGENT (CONTROL 1) (WITH LIGHT IRRADIATION)

X-axis: WAVELENGTH [nm]

Y-axis: FLUORESCENCE DETECTOR OUTPUT [counts]

# FIG. 13

TIME-DEPENDENT CHANGE IN FLUORESCENCE INTENSITY IN CARDIAC MUSCLE TISSUE
IMMERSED IN AGENT (EXAMPLE 1) AT WAVELENGTH OF 704nm

## FIG. 14

FLUORESCENCE SPECTRUM OF
TALAPORFIN SODIUM SOLUTION (CONTROL 2)

FLUORESCENCE DETECTOR OUTPUT [counts] vs. WAVELENGTH [nm]

# FIG. 15

SPECTROMETER  62′

64′

60′

63′  65′

45f′

44f′

30′ (44′,45′)

LIGHT SOURCE
633±2nm  61′

EP 3 067 092 A1

# FIG. 16

TRANSMISSION SPECTRA OF POLARIZATION BEAM SPLITTER 65'
AND DICHROIC MIRROR 63'

EP 3 067 092 A1

# FIG. 17

FLUORESCENCE SPECTRUM AT LIGHT IRRADIATION START TIME IN VERIFICATION TEST 2

# FIG. 18

FLUORESCENCE SPECTRUM AFTER 3 MINUTES
FROM LIGHT IRRADIATION START IN VERIFICATION TEST 2

# FIG. 19

### TIME-DEPENDENT CHANGE IN FLUORESCENCE INTENSITY AT WAVELENGTH OF 710nm IN VERIFICATION TEST 2

— THREE-INTERVAL MOVING AVERAGE (fluo.(710nm))

FLUORESCENCE DETECTOR OUTPUT [counts]

IRRADIATION TIME [s]

## INTERNATIONAL SEARCH REPORT

| | International application No. |
|---|---|
| | PCT/JP2014/079420 |

| A. CLASSIFICATION OF SUBJECT MATTER |
|---|
| *A61N5/067*(2006.01)i |

According to International Patent Classification (IPC) or to both national classification and IPC

| B. FIELDS SEARCHED |
|---|

Minimum documentation searched (classification system followed by classification symbols)
A61N5/00-5/10, A61B13/00-18/28, A61M25/00-25/18

Documentation searched other than minimum documentation to the extent that such documents are included in the fields searched
| Jitsuyo Shinan Koho | 1922–1996 | Jitsuyo Shinan Toroku Koho | 1996–2015 |
| Kokai Jitsuyo Shinan Koho | 1971–2015 | Toroku Jitsuyo Shinan Koho | 1994–2015 |

Electronic data base consulted during the international search (name of data base and, where practicable, search terms used)

| C. DOCUMENTS CONSIDERED TO BE RELEVANT |
|---|

| Category* | Citation of document, with indication, where appropriate, of the relevant passages | Relevant to claim No. |
|---|---|---|
| Y<br>A | JP 2012-510084 A (Vimecon GmbH),<br>26 April 2012 (26.04.2012),<br>paragraphs [0017] to [0024]; fig. 1 to 2<br>& WO 2010/060924 A1 & US 2011/0230941 A1 | 1-6,9-10<br>7-8 |
| Y | JP 2008-520363 A (Biosense Webster, Inc.),<br>19 June 2008 (19.06.2008),<br>paragraph [0017]; fig. 1<br>& US 2006/0122587 A1 & WO 2006/055733 A1 | 1-6,9-10 |
| Y | JP 2013-13726 A (Biosense Webster (Israel), Ltd.),<br>24 January 2013 (24.01.2013),<br>paragraphs [0020], [0027]; fig. 2, 6<br>& US 2013/0006238 A1 & EP 2540245 A1 | 5-6 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>09 January 2015 (09.01.15) | Date of mailing of the international search report<br>27 January 2015 (27.01.15) |
|---|---|
| Name and mailing address of the ISA/<br>Japan Patent Office | Authorized officer |
| Facsimile No. | Telephone No. |

Form PCT/ISA/210 (second sheet) (July 2009)

**INTERNATIONAL SEARCH REPORT**

| International application No. |
| --- |
| PCT/JP2014/079420 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | JP 2009-56290 A  (Biosense Webster, Inc.), 19 March 2009 (19.03.2009), paragraphs [0025], [0033], [0039]; fig. 4, 6 & US 2009/0005771 A1    & EP 2008602 A1 | 6 |
| Y | JP 2011-212423 A  (Sony Corp.), 27 October 2011 (27.10.2011), paragraphs [0051], [0052], [0054]; fig. 1 to 3 & US 2013/0123642 A1    & EP 2548616 A1 | 10 |
| A | US 2009/0275931 A1  (MARKUS Kai Ulf), 05 November 2009 (05.11.2009), entire text; all drawings & WO 2007/118745 A1 | 1-10 |
| A | JP 2005-512668 A  (AFX, Inc.), 12 May 2005 (12.05.2005), paragraphs [0072], [0100] to [0103]; fig. 9, 11, 14A to 14B & US 2002/0087151 A1    & WO 2003/053259 A2 | 1-10 |
| A | WO 2011/105631 A1  (Keio University), 01 September 2011 (01.09.2011), entire text; all drawings & US 2012/0330293 A1    & EP 2540247 A1 | 1-10 |
| A | JP 2008-501444 A  (Edwards Lifesciences Corp.), 24 January 2008 (24.01.2008), entire text; all drawings & US 2005/0273090 A1    & WO 2006/007305 A2 | 1-10 |
| E,A | JP 2014-221117 A  (Arai MedPhoton Research Laboratories, Corp.), 27 November 2014 (27.11.2014), entire text; all drawings (Family: none) | 1-10 |

Form PCT/ISA/210 (continuation of second sheet) (July 2009)

**EP 3 067 092 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012147937 A
- JP 2008501441 A
- JP 6080671 A
- JP 2961074 B